# EUROPÄISCHE PATENTSCHRIFT

(11) **EP 4 017 031 B1**
(45) Veröffentlichungstag und Bekanntmachung des Hinweises auf die Patenterteilung: **27.11.2024**
(21) Anmeldenummer: 22153552.9
(22) Anmeldetag: 07.07.2015
(51) Int. Cl.: H04R 25/00, A61B 5/12

(54) **HÖRSYSTEM MIT ANWENDERSPEZIFISCHER PROGRAMMIERUNG**
HEARING SYSTEM WITH USER-SPECIFIC PROGRAMMING
SYSTÈME AUDITIF À PROGRAMMATION SPÉCIFIQUE D'UN UTILISATEUR

(30) Priorität: 09.07.2014 CH 10472014
(43) Veröffentlichungstag der Anmeldung: 22.06.2022
(62) Teilanmeldung aus: 15736240.1
(73) Patentinhaber: KOJ hearing network GmbH, 77855 Achern (DE)
(72) Erfinder: KOJ, Andreas Thomas, 8052 Zürich (CH)
(74) Vertreter: Bittner, Peter

(56) Entgegenhaltungen:
- US-A- 4 637 402
- US-A1- 2007 003 077
- US-A1- 2012 051 569

## Beschreibung

### TECHNISCHES GEBIET

Die vorliegende Erfindung liegt im Bereich von Hörsystemen, insbesondere Hörgeräten und implantierbaren Hörsystemen sowie ihrer Programmierung. Die Erfindung betrifft ein Verfahren zur anwenderspezifischen Programmierung eines Hörsystems, insbesondere eines externen Hörgeräts oder eines implantierbaren Hörsystems. Die Erfindung betrifft ferner ein Hörsystemprogrammiergerät und eine Hörsystemanordnung.

### TECHNISCHER HINTERGRUND

Hörsysteme zur Verbesserung des Hörvermögens werden seit vielen Jahren in grosser Zahl eingesetzt und sind im Grundsatz bestens bekannt. Als übliche Bauformen werden insbesondere verschiedene Formen von Hinter-dem-Ohr-Geräten sowie Im-Ohr-Geräten eingesetzt, daneben aber auch weitere spezialisierte Systeme wie Knochenleitungshörgeräte und implantierbare Systeme, insbesondere implantierbare Hörgeräte und Cochleaimplantate.

Dem derzeitigen technischen Stand entsprechende Hörsysteme weisen häufig eine komplexe digitale Signalverarbeitung auf und sind in zahlreichen Parametern programmierbar, beispielsweise mittels Fernsteuerungen, spezieller Programmiergeräte und/oder PCs.

Aus WO 2005/12528 ist bekannt, einem Anwender verschiedene Töne zu präsentieren und die Programmierung der Verstärkung eines Hörsystems so anzupassen, dass die vom Anwender subjektiv wahrgenommene Lautstärke jeweils gleich ist.

Aus US 2010/0329490 ist bekannt, einem Anwender Testsignale mit jeweils unterschiedlichen Programmierungen bzw. Parametrisierungen zuzuführen und nach der jeweils subjektiv empfundenen besten Verständlichkeit bewerten zu lassen.

Aus US 2011/0249839 ist bekannt, die Verstärkung eines Hörsystems schrittweise zu erhöhen, wobei der Anwender selbst die Initiierung des jeweils nächsten Schritts auslöst.

Aus US2007/003077A1 ist bekannt, einem Anwender verschiedene Wörter für einen Konsonanten-Unterscheidungstest wiederholt zu präsentieren, auf dessen Basis das Hörgerät auch über einen längeren Zeitraum angepasst wird.

Aus US4637402A ist ein Verfahren zur quantitativen Messung des Hördefizits bekannt.

Aus US2012/051569A1 ist eine automatisierte Anpassung von Hörgeräten bekannt, welche eine Evaluierung der Sprachverständlichkeit umfasst.

### DARSTELLUNG DER ERFINDUNG

Ungeachtet des technischen Fortschritts im Bereich der Hörsysteme und ihrer aufwändigen und ausgefeilten Gestaltung ist die im Alltagseinsatz durch ein Hörsystem erzielte Verbesserung des Hörvermögens in vielen Fällen unbefriedigend bzw. mangelhaft. Häufig wird der Einsatz des Hörsystems auch abgebrochen. Ein wesentlicher Grund hierfür ist, dass sich das subjektive Hörvermögen in Alltagssituationen trotz der durch das Hörsystem realisierten Verstärkung nicht oder nur geringfügig verbessert, während die subjektive Wahrnehmung von Lärm und Störgeräuschen aufgrund der Verstärkung durch das Hörsystem ansteigt. Die Möglichkeiten, diesen Problemen mit Mitteln des Hörsystemdesigns und insbesondere der (digitalen) Signalverarbeitung entgegenzuwirken, sind beschränkt und nach derzeitigem Stand insgesamt unzureichend.

Die vorliegende Erfindung ist vor folgendem Hintergrund zu sehen: Während ein Hörsystem, z. B. ein Hörgerät bekannter Bauart, auf rein akustischer und physikalischer Ebene wirkt und den das Trommelfell beaufschlagenden Schalldruck erhöht, ist für das (Sprach-)verstehen und weitere mit dem Hören verbundene Wahrnehmungsprozesse (z. B. die Richtungslokalisation) die nachfolgende Verarbeitung im Gehirn von zentraler Bedeutung, welche weitgehend erlernt ist. Grundsätzlich ist das Gehirn einer Person mit gesundem bzw. ungeschädigten Hörvermögen im Zusammenspiel mit einem ungeschädigten Gehör insbesondere in der Lage, sich auf bestimmte interessierende Schallereignisse (z. B. eine flüsternde oder von starken Störgeräuschen überlagerte Stimme) zu konzentrieren und sonstigen Schallereignisse und Schallsignale auszublenden bzw. zu maskieren.

Mit einsetzendem und fortschreitendem Hörverlust verliert das Gehirn jedoch diese Fähigkeit zur akustischen "Konzentration" weitgehend; die Fähigkeit zur Unterscheidung von interessierenden akustischen Nutzsignalen zu sonstigen Störsignalen geht verloren. Der anschliessende Einsatz eines Hörsystems, beispielsweise eines Hörgerätes, führt daher in vielen Fällen subjektiv im Wesentlichen zur Wahrnehmung von mehr Lärm, nicht aber zu einer befriedigenden Verbesserung des Hörvermögens.

Es ist eine Aufgabe der vorliegenden Erfindung, die Situation bei der Anwendung von Hörsystemen zu verbessern und insbesondere die Nachteile des Standes der Technik zumindest teilweise zu vermeiden. Diese Aufgabe wird gelöst durch ein Verfahren zur patientenspezifischen Programmierung eines Hörsystems nach Anspruch 1, ein Hörsystemprogrammiergerät nach Anpsruch 6 sowie ein Computerprogrammprodukt nach Anspruch 7. Spezielle vorteilhafte Ausgestaltungen werden durch die abhängigen Patentansprüche sowie die Beschreibung und die Figuren definiert.

Ein Verfahren zur anwenderspezifischen Programmierung eines Hörsystems umfasst die folgenden Schritte:
a) Bereitstellen eines Hörsystemprogrammiergeräts und des Hörsystems;
b) Durchführen mindestens einer Datensammelsequenz, die mindestens eine Datensammelsequenz umfassend:
   - Erzeugen und Abstrahlen mindestens eines akustischen Testsignals und
   - Empfangen mindestens einer Rückmeldung eines Anwenders des Hörsystems in Reaktion auf das mindestens eine Testsignal durch das Hörsystemprogrammiergerät;
c) Durchführend mindestens einer Programmiersequenz, die mindestens eine Programmiersequenz umfassend:
   - Bestimmen einer modifizierten Programmierung des Hörsystems durch das Hörsystemprogrammiergerät, wobei das Bestimmen der modifizierten Programmierung unter Berücksichtigung einer Auswertung der empfangenen mindestens einen Rückmeldung des Anwenders des Hörsystems erfolgt;
   - Übertragen der modifizierten Programmierung vom Hörsystemprogrammiergerät zum Hörsystem mittels einer Datenkommunikationsverbindung zwischen Hörsystemprogrammiergerät und Hörsystem;
   - Ändern der Programmierung des Hörsystems zur modifizierten Programmierung.

Das Erzeugen des mindestens einen akustischen Testsignals erfolgt durch das Hörsystemprogrammiergerät. Das Abstrahlen kann ebenfalls durch das Hörsystemprogrammiergerät erfolgen. In anderen Ausführungsformen kann das Abstrahlen des mindestens einen akustischen Testsignals aber auch durch eine weitere externe Wiedergabeeinheit erfolgen. Eine solche Wiedergabeeinheit kann insbesondere durch das zu Programmierende Hörsystem selbst gebildet werden, wie weiter unten näher erläutert. In derartigen Ausführungsformen umfasst das Verfahren ein Übertragen des mindestens einen Testsignals vom Hörsystemprogrammiergerät an die externe Wiedergabeeinheit.

Das Hörsystem kann von grundsätzlich jeder bekannten Bauart und insbesondere ein Hinter-dem-Ohr-Gerät oder Im-Ohr-Gerät sein. Es kann aber ebenfalls ein Knochenleitungshörgerät oder ein implantierbares System, insbesondere ein implantierbares Hörgerät oder ein Cochleaimplantat sein. Das Hörsystem kann ferner zwei grundsätzlich separate Geräte umfassen, die jeweils einem Ohr des Anwenders zugeordnet sind.

Der Benutzer bzw. Träger des Hörsystems wird in diesem Dokument grundsätzlich als "Anwender" bezeichnet. Bei diesem Anwender handelt es sich häufig um einen Menschen mit eingeschränktem Hörvermögen bzw. mit teilweisem Hörverlust. Der Anwender kann aber auch ein Mensch mit einem sonstigen Leiden sein, welches das Hörvermögen beeinträchtigt, beispielsweise einem Tinnitus, einer Überempfindlichkeit, einer Konzentrationsschwäche und/oder weiteren Defiziten in der auditiven Verarbeitung, beispielsweise einem Aufmerksamkeitsdefizitsyndrom (ADS), einem Aufmerksamkeitsdefizit-/Hyperaktivitätssyndrom (ADHS), oder einer auditiven Verarbeitungs- und Wahrnehmungsstörung (AVWS).

Wie weiter unten im Zusammenhang von Ausführungsbeispielen beschrieben, liefert das mindestens eine Testsignal in Verbindung mit der Auswertung der Rückmeldung des Anwenders eine Datenbasis für die Modifikation der Programmierung des Hörsystems. Daneben kann das Testsignal in einigen Ausführungsformen als Trainingssignal dienen, mit dessen Hilfe das Gehirn des Anwenders hinsichtlich seiner Fähigkeit trainiert wird, Schallereignisse korrekt zu bewerten und einzuordnen und insbesondere zwischen Nutzsignalen und Störsignalen zu unterscheiden. Es wird ein Verfahren zur patientenspezifischen Programmierung eines Hörsystems bereitgestellt, bei dem sich die Programmierung an das Hörvermögen und Hörverständnis des Anwenders adaptiv anpasst. Wie nachfolgend dargestellt, kann diese Adaption insbesondere über einen längeren Zeitraum fortlaufend bzw. verteilt erfolgen, wobei Änderungen in diesem Zeitraum erfolgende Änderungen im Hörvermögen bei der Programmierung des Hörsystems berücksichtigt werden.

Die "Programmierung" des Hörsystems umfasst die Gesamtheit aller zur patientenspezifischen Anpassung des Hörsystems einstellbarer oder auswählbarer Parameter, wie globale (frequenzunspezifische) Verstärkungen, frequenzabhängige oder frequenzspezifische Verstärkungen, zusätzliche Filter oder Kompressionsparameter einer Dynamikkompression im Hörsystem, Parameter von Limitern (Begrenzern) sowie weiteren in einigen Hörsystemen vorgesehenen Funktionen, wie eine Mikrofonauswahl.

Die Programmierung im Kontext der vorliegenden Erfindung kann sich je nach konkreter Situation zusätzlich oder alternativ auch auf andere oder weitere der programmierbaren Parameter beziehen.

In einigen Ausführungsformen umfasst das Auswerten der mindestens einen Rückmeldung des Anwenders des Hörsystems ein Vergleichen der mindestens einen Rückmeldung mit einer entsprechenden Referenz-Rückmeldung. Die Bestimmung der Modifikation der Programmierung basiert dann mindestens teilweise auf dem Ergebnis des Vergleichs. Die Referenz-Antworten können z. B. online von einer externen Datenbank abgefragt werden oder in einem Speicher des Hörsystemprogrammiergeräts gespeichert werden, z. B. in Form einer Datenbank.

Wie weiter unten näherer dargestellt, kann die Referenz-Rückmeldung dem korrekten Lösen einer (akustischen) Übungsaufgabe oder allgemein dem korrekten Erkennen einer akustischen Situation entsprechen, und weitere alternative Rückmeldungen einem nicht korrekten (d. h. falschen) Lösungen einer Übungsaufgabe bzw. dem nicht korrekten einer akustischen Situation. Dabei kann die Rückmeldung des Anwenders als Antwort auf eine durch das Testsignal bestimmte Frage aufgefasst werden. Die Rückmeldung des Anwenders wird demnach binär entweder als "korrekt" oder nicht korrekt" klassifiziert. Hier deuten insbesondere nicht korrekte, d. h. nicht der Referenz-Rückmeldungen entsprechende Rückmeldungen, auf ein Erfordernis für eine Änderung der Programmierung, beispielsweise der Verstärkung. Eine korrekte Rückmeldung bzw. eine korrekte Antwort deuten dagegen darauf hin, dass hinsichtlich des durch die jeweilige Übungsaufgabe abgedeckten hörakustischen Aspekts kein Erfordernis für eine Modifizierung der Programmierung des Hörsystems besteht.

In der Praxis kann anstelle eines Vergleichs einer einzelnen Rückmeldung mit einer einzelnen Referenz-Rückmeldung eine Mehrzahl von Rückmeldungen mit einer entsprechenden Mehrzahl von Referenz-Rückmeldungen verglichen und für die Bestimmung der modifizierten Programmierung berücksichtigt werden, wobei jedes Abstrahlen eines Testsignals und das Empfangen einer zugehörigen Rückmeldung dem Lösen einer einzelnen Übungsaufgabe durch den Anwender entspricht. Eine Modifikation der Programmierung kann z. B. dann erfolgen, wenn - für eine Reihe gleicher bzw. ähnlicher Übungsaufgaben bzw. Testsignale, der Anteil der nicht-korrekten Antworten (nicht der jeweiligen Referenz-Rückmeldung entsprechenden Rückmeldungen) einen Schwellwert überschreitet. Wie weiter untern näher dargestellt, können die einzelnen Übungsaufgaben insbesondere auch zeitlich verteilt, z. B. über einen Zeitraum von Tagen bis Wochen absolviert werden.

Das hier vorgeschlagene Vorgehen hat gegenüber aus dem Stand der Technik bekannten Ansätzen den Vorteil, dass eine Modifikation in der Programmierung des Hörsystems aufgrund objektiver und nachvollziehbarer Kriterien erfolgt, und nicht in erster Linie auf der Grundlage einer subjektiven Bewertung der Programmierung durch den Anwender. Dadurch, dass die Referenz-Rückmeldungen (und somit die korrekten Antworten auf die Übungsaufgaben) im Hörsystemprogrammiergerät vorliegen und der Vergleich durch das Hörsystemprogrammiergerät erfolgt, ist es ferner nicht erforderlich, dass der Anwender das Testsignal zuvor kennt. Stattdessen wird er - entsprechend im Alltag typischerweise auftretenden Situationen - mit ihm in ihrer konkreten Ausgestaltung unbekannten Schallereignissen konfrontiert. Die im Stand der Technik bekannten Lösungen beruhen darauf, dass dem Anwender dasselbe Testsignal mit unterschiedlicher Programmierung des Hörsystems präsentiert wird und er subjektiv über die beste Variante entscheidet. In einigen Ausführungsformen umfasst das mindestens eine Testsignal mindestens eine Zufallskomponente. Eine Zufallskomponente kann z. B. mittels eines Zufallszahlengenerators realisiert werden und sich beispielsweise auf eine oder mehrere der folgenden Aspekte beziehen: Auswahl eines Testsignals; Zusammensetzung des Testsignals aus Nutzsignal und Störsignal wie nachfolgend beschrieben; Lautstärke des Testsignals; Stimme von gesprochenen Bestandteilen des Testsignals, Richtung, aus der das Testsignal kommt; Frequenz bzw. Tonhöhe des Testsignals; Musikinstrument, mit dem eine Tonfolge oder Melodie wiedergegeben wird; Dauer der Präsentation bzw. Abstrahlung des Testsignals.

In einigen Ausführungsformen umfasst das mindestens eine Testsignal ein gestörtes Testsignal, wobei das gestörte Testsignal ein Nutzsignal und ein Störsignal umfasst. In einigen Ausführungsformen mit gestörtem Testsignal umfasst das Störsignal ein gleichförmiges Störsignal und/oder mindestens ein Impulssignal. Die Störsignale können sowohl durch das Hörsystemprogrammiergerät synthetisiert werden, beispielsweise durch einen Hard- und/oder softwaremässig realisierten Rauschgenerator, als auch durch gespeicherte reale Störgeräusche sein, mit denen ein Anwender im Alltag typischerweise konfrontiert ist.

In einigen Ausführungsformen mit gestörtem Testsignal umfasst das Verfahren ein Erhöhen und/oder Erniedrigen eines Pegels des Störsignals relativ zum Nutzsignal in Abhängigkeit der empfangenen mindestens einen Rückmeldung des Anwenders des Hörsystems. Insbesondere kann eine durch den Anwender gegebene Rückmeldung alternativ einer korrekten oder nicht korrekten Lösung einer vorbestimmten Übungsaufgabe entsprechen. Dann kann eine Erhöhung des Pegels des Störsignals bei einem hohen Anteil korrekter Antworten erfolgen, wodurch der Anwender ohne Überforderung allmählich an akustisch schwierigere Situationen gewöhnt wird. Bei einem geringen Anteil korrekter Antworten kann der Pegel des Störsignals entsprechend reduziert werden oder eine weitere Erhöhung des Störsignalpegels unterlassen oder zumindest verlangsamt werden. Die Abstimmung des Verhältnisses zwischen Störsignal und Nutzsignal kann einheitlich für alle Übungsaufgaben und Testsignale erfolgen, oder für verschiedene Typen von Übungsaufgaben und Testsignalen separat und adaptiv. So kann für akustische Situationen, in denen der Anwender (noch) relativ viele Fehler macht, z.B. generell ein geringerer Störpegel gewählt werden als für Übungsaufgaben und Testsignale, in denen der Anwender weitestgehend oder vollständig korrekt antwortet, d. h. die Rückmeldung jeweils der Referenz-Rückmeldung entspricht.

In einigen Ausführungsformen umfasst das mindestens eine Testsignal mindestens eines von: gesprochenen Ziffern, Zahlen, Lauten, Silben Phonemen, Wörtern, Wortgruppen, Sätzen, Tönen, Klängen und Tonfolgen oder Melodien. Grundsätzlich kann ein Testsignal bzw. ein Nutzsignal als Bestandteil des Testsignals derartige akustische Signale umfassen, die im Alltag durch den Anwender des Hörsystems wahrgenommen und verarbeitet werden soll, insbesondere Sprache und ihre Bestandteile.

In einigen Ausführungsformen umfasst das Verfahren das Durchführen mehrerer Datensammelsequenzen, wobei sich das jeweils erzeugte mindestens eine Testsignal zwischen den einzelnen Datensammelsequenzen mindestens für einen Teil der Datensammelsequenzen unterscheidet.

In einigen Ausführungsformen mit mehreren Datensammelsequenzen umfasst das Verfahren das Durchführen einer Anzahl von Datensammelsequenzen über mehrere Tage, insbesondere über mehrere aufeinander folgenden Tage. An jedem Tage kann dabei eine oder wiederum eine Abfolge von Datensammelsequenzen durchgeführt werden. Ferner können die Schritte der Auswertung der Benutzerrückmeldung(en), des Bestimmens einer modifizierten Programmierung des Hörsystems sowie des Modifizierens der Programmierung des Hörsystems mehrmals während eines Tages, an jedem der Tage oder nur an einem Teil der Tage durchgeführt werden.

In einigen Ausführungsformen umfasst die Bestimmung der modifizierten Programmierung des Hörsystems eine Bestimmung einer modifizierten Verstärkung. Die Verstärkung wird hier insbesondere als Funktion der Frequenz betrachtet, ist also im Allgemeinen für verschiedene Frequenzen verschieden.

In einigen Ausführungsformen mit einer Änderung der Verstärkung umfasst die Bestimmung der modifizierten Verstärkung die Bestimmung einer frequenzabhängigen Änderung der Verstärkung in Abhängigkeit der mindestens einen Rückmeldung des Anwenders. Eine "frequenzabhängige Änderung der Verstärkung" meint insbesondere die gezielte selektive Änderung der Verstärkung für eine oder mehrere bestimmte Frequenz(en) beziehungsweise Frequenzbereich(e), während die Verstärkung für die übrigen Frequenzen bzw. Frequenzbereiche unverändert bleibt. Die Bestimmung der modifizierten Verstärkung kann insbesondere in Abhängigkeit von und unter Berücksichtigung der mindestens einen Rückmeldung durch den Anwender erfolgen. Wie weiter unten im Zusammenhang beispielhafter Ausführungsformen näher dargestellt, erlaubt eine derartige Ausführungsform insbesondere eine gezielte Abstimmung der Programmierung des Hörsystems dahin gehend, dass ähnlich klingende und damit in ihrer Differenzierung kritische Schallereignisse, insbesondere Konsonanten und allgemein Phoneme, durch den Anwender zumindest befriedigend gut differenziert werden können.

In einigen Ausführungsformen mit einer Änderung der Verstärkung umfasst die Bestimmung der modifizierten Verstärkung die Bestimmung einer frequenzunabhängigen Änderung der Verstärkung in Abhängigkeit der mindestens einen Rückmeldung des Anwenders umfasst. Eine "frequenzunabhängige Änderung der Verstärkung" ist eine Änderung der Verstärkung, die den vom Hörsystem verarbeitbaren akustischen Bereich insgesamt und im Wesentlichen in gleicher Weise betrifft, beispielsweise eine Anhebung der Verstärkung um eine vorgegebene Zahl von Dezibel über den gesamten Frequenzbereich. Eine frequenzunabhängige Änderung der Verstärkung kann basierend auf Rückmeldungen des Anwenders auf Testsignale wie zuvor beschrieben umfassen, aber auch unabhängig davon z. B. zeitgesteuert erfolgen.

In einigen derartigen Ausführungsformen umfasst das Verfahren die Durchführung mehrerer Programmiersequenzen mit einer schrittweisen Anhebung der Verstärkung in Richtung einer Zielverstärkung. Bei einer über einen Zeitraum von mehreren Tagen verteilten Durchführung des Verfahrens, wie zuvor Beschreibung, können einzelne Schritte der schrittweisen Anhebung der Verstärkung, insbesondere der frequenzunabhängigen Verstärkung, an verschiedenen Tagen erfolgen. Wie im Zusammenhang exemplarischer Ausführungsbeispiele erläutert, kann das Verfahren auch eine Verringerung bzw. Absenkung der Verstärkung und/oder ein Beibehalten der Verstärkung ohne Modifikation umfassen. Bei einer über einen Zeitraum von mehreren Tagen verteilten Ausführung des Verfahrens kann etwa an einigen Tagen die bisherige Verstärkung unverändert beibehalten oder abgesenkt werden.

In einigen derartigen Ausführungsformen umfasst das Verfahren das Durchführen einer ersten Programmierstufe und das nachfolgende Durchführen einer zweiten Programmierstufe. Jede der ersten und der zweiten Programmierstufe umfasst dabei das Durchführen mindestens einer Datensammelsequenz. Die Bestimmung der modifizierten Verstärkung erfolgt in der ersten Programmierstufe frequenzunabhängig. In der zweiten Programmierstufe erfolgt die Bestimmung der modifizierten Verstärkung frequenzabhängig. In einigen derartigen Ausführungsformen erfolgt die Durchführung jeder der ersten und der zweiten Programmierstufe verteilt über mehrere Tage.

Ein Hörsystemprogrammiergerät umfasst:
a) einen zur Erzeugung mindestens eines akustischen Testsignals ausgelegten Testsignalgenerator;
b) mindestens eine mit dem Testsignalgenerator operativ gekoppelte und zur akustischen Abstrahlung des mindestens einen Testsignals ausgelegte akustische Widergabeeinheit und/oder eine mit dem Testsignalgenerator operativ gekoppelte Übertragungseinheit zur Übertragung des mindestens einen Testsignals an eine externe Wiedergabeeinheit;
c) eine zum Empfang einer Reaktion eines Benutzers in Reaktion auf das mindestens eine Testsignal ausgelegte Eingabeeinheit;
d) eine zum Bestimmen einer modifizierten Programmierung eines Hörsystems unter Berücksichtigung der empfangenen Rückmeldung ausgelegte Programm-Modifikationseinheit;
e) einen mit der Programm-Modifikationseinheit operativ gekoppelten lernfähigen Konfigurationsspeicher zur Speicherung einer Konfiguration der Programmierung des Hörsystems;
f) eine zur Datenkommunikation mit dem Hörsystem und zur Übertragung der modifizierten Programmierung an das Hörsystem ausgelegte Kommunikationseinheit.

Das Hörsystemprogrammiergerät kann insbesondere zur Durchführung eines erfindungsgemässen Verfahrens zur anwenderspezifischen Programmierung eines Hörsystems ausgelegt sein. Somit wird ein Hörsystemprogrammiergerät bereitgestellt, welches eine patientenspezifische Programmierung eines Hörsystems derart gestattet, dass sich die Programmierung an das sich ändernde Hörvermögen des Anwenders adaptiert.

In einer Ausführungsform ist die Programm-Modifikationseinheit dazu ausgelegt, ein Vergleichen der mindestens einen Rückmeldung mit einer entsprechenden Referenz-Rückmeldung vorzunehmen und die modifizierte Programmierung mindestens teilweise basierend auf dem Ergebnis des Vergleichs zu bestimmen.

Die genannten funktionellen Komponenten des Hörsystems können strukturell in einem einzelnen oder verteilt auf verschiedene Geräte realisiert werden.

Somit offenbaren beschriebene Ausführungsformen und Varianten des Verfahrens zur anwenderspezifischen Programmierung eines Hörsystems zugleich entsprechende Ausführungsformen eines Hörsystemprogrammiergeräts. In analoger Weise offenbaren Ausführungsformen des Hörsystemprogrammiergeräts zugleich entsprechende Ausführungsformen des Verfahrens zur Programmierung eines Hörsystems.

Eine Hörsystemanordnung umfasst:
a) ein Hörsystemprogrammiergerät wie zuvor und nachfolgend beschrieben;
b) ein programmierbares Hörsystem, wobei das Hörsystem eine zur Datenkommunikation mit der Kommunikationseinheit des Hörsystemprogrammiergeräts ausgelegte Kommunikationsschnittstelle aufweist und zur Änderung seiner Programmierung mittels über die Kommunikationsschnittstelle des Hörsystems empfangenen Daten ausgelegt ist.

Ebenso wie ein erfindungsgemässes Hörgerätprogrammiergerät kann eine erfindungsgemässe Hörsystemanordnung strukturell verteilt realisiert werden.

### KURZE BESCHREIBUNG DER FIGUREN

- Figur 1: zeigt eine beispielhafte Hörsystemanordnung mit einem Hörsystem und einem Hörsystemprogrammiergerät in einer schematischen strukturellen und funktionellen Ansicht;
- Figur 2: zeigt eine beispielhafte äussere Gestaltung eines Hörsystemprogrammiergeräts in schematischer Ansicht;
- Figur 3: zeigt schematisch eine mögliche Gliederung des Ablaufs eines Verfahrens zur patientenspezifischen Programmierung eines Hörsystems;
- Figur 4: zeigt schematisch den Ablauf eines Verfahrens zur patientenspezifischen Programmierung eines Hörsystems;
- Figur 5: zeigt in schematischer Weise den Ablauf eines Teils des Verfahrens zur patientenspezifischen Programmierung eines Hörsystems gemäss Figur 4;
- Figur 6: zeigt schematisch eine frequenzunabhängige Änderung der Verstärkung des Hörsystems über die Zeit;
- Figur 7: zeigt schematisch die Umrisse von Spektrogrammen für zwei beispielhafte ähnliche Laute;
- Figur 8: zeigt schematisch den Ablauf einer frequenzabhängigen Änderung der Verstärkung des Hörsystems.

### AUSFÜHRUNGSBEISPIELE

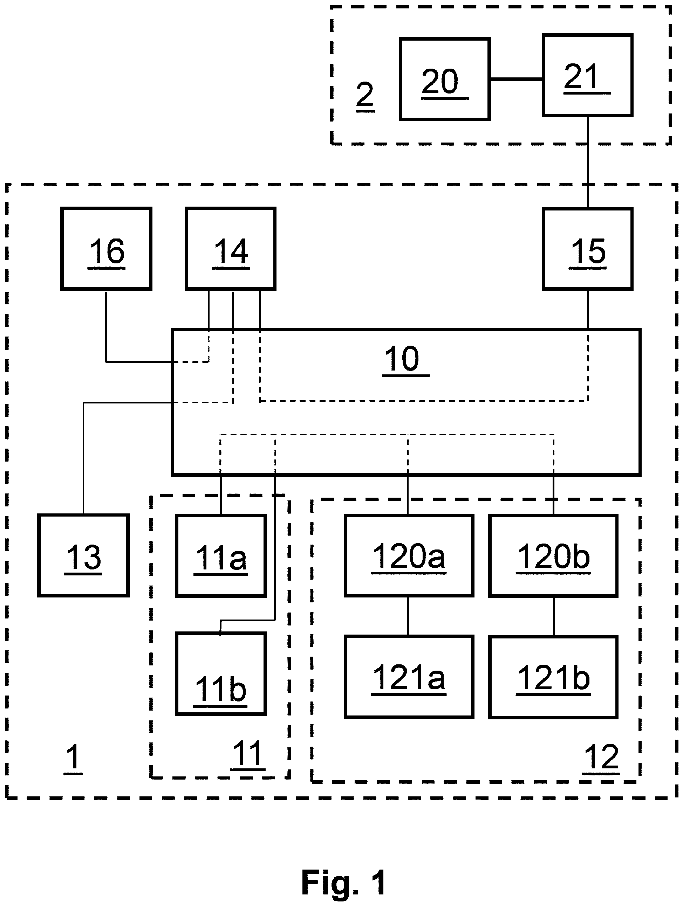

Figur 1 zeigt eine Hörsystemanordnung in einer schematischen strukturellen und funktionellen Ansicht. Die Hörsystemanordnung umfasst das Hörsystemprogrammiergerät 1 sowie das Hörsystem 2. Das Hörsystem 2 ist exemplarisch als externes Hörgerät, zum Beispiel als Hinter-dem-Ohr-Gerät oder Im-Ohr-Gerät grundsätzlich bekannter Bauweise angenommen, kann aber auch eine andere Art von Hörsystem entsprechend der allgemeinen Beschreibung sein. Ferner kann das Hörsystem 2 durch zwei getrennte Geräte gebildet werden, zum Beispiel durch zwei externe Hörgeräte der zuvor beschriebenen Art, von denen jedes einem der Ohren des Anwenders zugeordnet ist.

Die grundsätzliche Funktionalität des Hörsystems 2 ist in einer funktionalen Einheit 20 zusammengefasst dargestellt. Diese Einheit 20 ist operativ mit einer bidirektionale Kommunikationsschnittstelle 21 gekoppelt.

Das Hörsystemprogrammiergerät 1 umfasst eine zentrale Steuereinheit 10, einen Testsignalgenerator 11, eine akustische Wiedergabeeinheit 12, eine Eingabe- und Anzeigeeinheit 13, eine Programm-Modifikationseinheit 14, einen lernfähigen Konfigurationsspeicher 16 sowie eine bidirektionale Kommunikationseinheit 15, welche zur Datenkommunikation mit der Kommunikationseinheit 21 des Hörsystems 2 ausgelegt ist.

Die zentrale Steuereinheit 10 stellt die zentrale Kontrollinstanz des Hörsystemprogrammiergeräts 1 dar. Die zentrale Steuereinheit 10 steuert und koordiniert den Ablauf der von dem Hörsystemprogrammiergerät 1 durchgeführten Schritte und Funktionen. Die übrigen funktionellen Komponenten des Hörsystemprogrammiergeräts 1 sind mit der zentralen Steuereinheit 10 operativ gekoppelt. In nachfolgend näher beschriebenen Ausführungsformen, bei denen ein Verfahren zur anwenderspezifischen Programmierung des Hörsystems 2 zeitlich verteilt ausgeführt wird, wobei bei der Durchführung des Verfahrens nach vom Anwender zu absolvierenden Übungen, Aufgaben und Lektionen gegliedert ist, steuert die zentrale Steuereinheit 10 insbesondere die Abfolge der Übungen, Aufgaben und Lektionen, und damit den Gesamtablauf des Verfahrens.

Der Testsignalgenerator 11 umfasst einen Nutzsignalgenerator 11a sowie einen Störsignalgenerator 11b. Der Testsignalgenerator 11 stellt die erforderlichen Testsignale mit ihren jeweiligen Nutzensignalen und Störsignalen bereit. Nutzsignale wie auch Störsignale können durch den Nutzsignalgenerator 11a bzw. den Störsignalgenerator 11b in Form aufgenommener und in digital Form abgespeicherter akustischer Signale bereitstellen, beispielsweise in Form nach bekannten Verfahren der Tonaufnahmetechnik aufgenommener Sprache, Hintergrundgeräusche, usw. Die Verwendung von Aufzeichnungen "echter" akustischer Signale hat den Vorteil eines besonders naturgetreuen Klangs, der, zum Beispiel bei Sprache, mit einer realen menschlichen Stimme identisch ist. Alternativ oder zusätzlich können der Nutzsignalgenerator 11a und/oder der Störsignalgenerator 11b des Testsignalgenerators 11 aber auch zur Synthese künstlicher Testsignale oder Teilen von Testsignalen ausgelegt sein. Hierzu kann der Nutzsignalgenerator 11a zum Beispiel eine Anordnung zur Sprachsynthese und der Störsignalgenerator 11b Impulsgeneratoren und Rauschgeneratoren grundsätzlich bekannter Art umfassen.

Die akustische Wiedergabeeinheit 12 ist beispielhaft zweikanalig und damit zur akustischen Wiedergabe und Abstrahlung von Stereosignalen ausgelegt. Die akustische Wiedergabeeinheit 12 umfasst, jeweils für den linken bzw. rechten Kanal, einen Verstärker 120a bzw. 120b sowie einen Lautsprecher 121a bzw. 121b. Die Verstärker 120a, 120b sowie die Lautsprecher 121a, 121b sind in grundsätzlich bekannter Weise aufgebaut und können bedarfsweise auch mehrere Wege für unterschiedliche Frequenzen zuzüglich Frequenzweichen aufweisen. Bevorzugt umfasst die akustische Wiedergabeeinheit 12 ferner (in Figur 1 nicht separat dargestellte) digital-nach-analog-Wandler zur Wandlung der in digitaler Form bereitgestellten Testsignale.

In alternativen Ausführungsformen umfasst das Hörsystemprogrammiergerät keine akustische Wiedergabeeinheit 12. Stattdessen werden Testsignale mittels einer Übertragungseinheit, welche durch die weiter unten näher ausgeführte Kommunikationsschnittstelle 15 und/oder eine weitere Schnittstelle gebildet werden kann, unmittelbar an das Hörsystem 2 übertragen durch dieses wiedergegeben.

Die Eingabe- und Anzeigeeinheit 13 ist hier beispielhaft als Touchscreen angenommen. Sie kann aber zusätzlich oder alternativ auch weitere Einrichtungen, wie eine Maus, eine konventionelle Tastatur, eine Spracheingabeeinheit mit Mikrofon, eine kameragestützte Vorrichtung zur Erkennung und Auswertung von Handbewegungen und/oder Gesten und/oder Gesichtszügen usw. umfassen.

Die Kommunikationseinheit 15 zur bidirektionalen Datenkommunikation mit der Kommunikationseinheit 21 des Hörsystems 2 ist in grundsätzlich bekannter Weise ausgeführt, beispielsweise nach dem Bluetooth-Standard oder als NFC-Einheit (Nahfeld-Kommunikationseinheit) Sie kann aber beispielsweise auch ein WLAN-Modul, eine Infrarot-Schnittstelle oder eine galvanische Schnittstelle mit elektrischen Kontakten umfassen.

Funktionelle Bestandteile und die Arbeitsweise der Programm-Modifikationseinheit 14 sowie des lernfähigen Konfigurationsspeichers 16 wird nachfolgend anhand der Funktion der Hörsystemanordnung näher erläutert.

Die Darstellung gemäss Figur 1 dient vor allem einer Übersicht über das Zusammenwirken der einzelnen funktionellen Bestandteile der Hörsystemanordnung. Sie impliziert keine spezielle, limitierende technische Realisierung. So können in Figur 1 separat dargestellte funktionelle Komponenten in einer konkreten technischen Ausführung ganz oder teilweise integriert sein. Ebenso kann eine einzelne funktionelle Komponente durch eine Mehrzahl struktureller Komponenten realisiert werden. Die Realisierung des Hörsystemprogrammiergeräts 1 erfolgt ferner typischerweise in einer Mischung von Hardwarekomponenten sowie Soft- und/oder Firmwarekomponenten, welche in einem nichtflüchtigen Speicher gespeichert sind. Zentraler Bestandteil des Hörsystemprogrammiergeräts 1 ist in typischen Ausführungsformen eine mittels Software und/oder Firmware entsprechend programmierte Computereinheit mit grundsätzlich bekannten Bestandteilen. Diese kann weitere Komponenten, z. B. den Touchscreen als Eingabe- und Anzeigeeinheit 13, bereitstellen und mittels entsprechender Programmierung insbesondere die zentrale Steuereinheit 10 und die Programm-Modifikationseinheit 14 sowie den lernfähigen Konfigurationsspeicher 16 realisieren. Selbstverständlich sind auch andere Ausführungen möglich, bei denen mehr oder alle funktionellen Komponenten durch entsprechend spezialisierte Hardware gebildet werden. Die in Figur 1 durch entsprechende Verbindungen und Signalpfade zwischen einzelnen funktionellen Einheiten bzw. Komponenten des Hörgeräteprogrammiergerätes 1 verdeutlichen exemplarische operative Kopplungen zwischen den jeweiligen Kopplungen bzw. Komponenten im gezeigten Ausführungsbeispiel, ohne zusätzliche Kopplungen oder alternative Realisierungen auszuschliessen.

In der in Figur 1 dargestellten Ausführungsform ist die Hörsystemanordnung strukturell und funktionell in sich abgeschlossen erfordert führ ihre Funktion insbesondere Datenaustausch mit weiteren externen Geräten, Vorrichtungen oder Systembestandteilen, wie externen Computern, Datenbanken oder Servern.

In weiteren ebenfalls möglichen Ausführungsformen ist das Hörsystemprogrammiergerät in seiner Funktionalität verteilt realisiert, wobei einzelne funktionelle Bestandteile z. B. in Form einer externen Einheit, die z. B. externe Server und/oder Datenbanken realisiert sind. Eine lokale Einheit umfasst dann typischerweise die Kommunikationsschnittstelle 21, die akustische Wiedergabeeinheit 12 sowie die Eingabe- und Anzeigeeinheit 13. Weitere Bestandteile können ganz oder teilweise in die externe Einheit ausgelagert sein. Die externe und die lokale Einheit kommunizieren dabei im Betrieb über entsprechende grundsätzlich bekannte Kommunikationsschnittstellen und Kommunikationskanäle, wie beispielsweise eine Internetverbindung. Eine derartige Architektur gestattet es, insbesondere Daten- und oder rechenintensive Funktionen ganz oder teilweise an die externe Einheit auszulagern. Hierdurch kann die lokale Einheit vergleichsweise kompakt, technisch wenig komplex und kostengünstig gestaltet werden. Die externe Einheit kann z. B. Datenbanken bzw. Bibliotheken von Testsignalen umfassen, welche bedarfsweise ganz oder teilweise an die lokale Einheit übermittelt werden. Ebenso kann die externe Einheit Funktionalitäten zur Modifikation der Programmierung des Hörsystems 2 bereitstellen, wie in Form von Programmcode gespeicherte Modifikationsalgorithmen, Listen und Look-Up-Tafeln.

Ebenso kann ein Gesamtablauf aus Aufgaben, Übungen und Lektionen, wie zuvor dargestellt und nachfolgend beispielhaft beschrieben, in einem Speicher der zentralen Steuereinheit 10 dauerhaft gespeichert oder ganz oder teilweise von einer externen Einheit übermittelt werden.

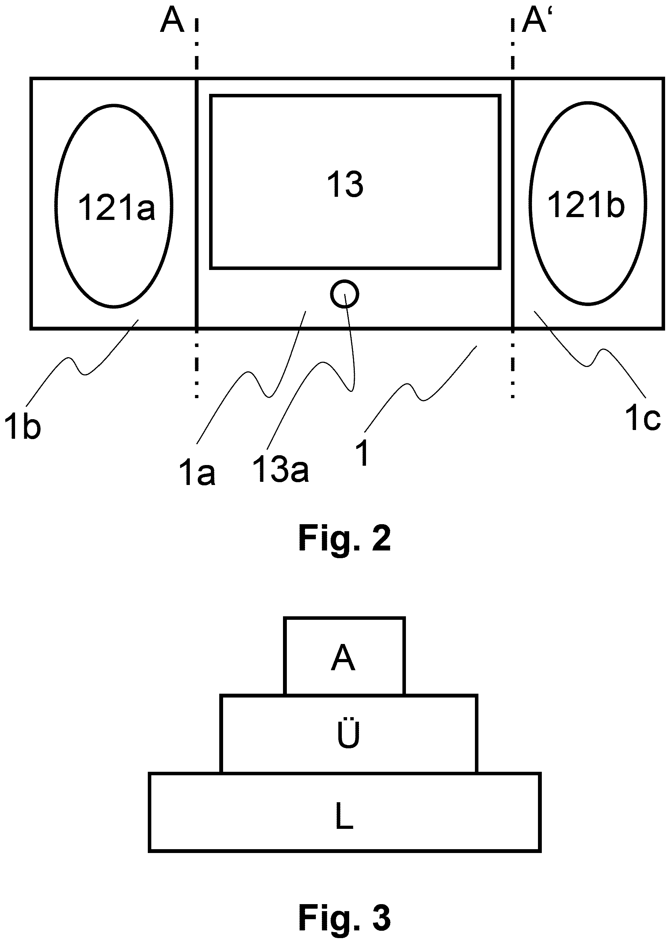

Nachfolgend wird zusätzlich auf Figur 2 Bezug genommen. Figur 2 zeigt eine mögliche äussere Gestalt eines Hörsystemprogrammiergeräts 1 gemäss Figur 1 in einer schematischen Aufsicht. Die Bestandteile des Hörsystemprogrammiergeräts 1 sind auf drei Module 1a, 1b, 1c mit jeweils einem eigenen Modulgehäuse aufgeteilt. Das Hörsystemprogrammiergerät 1 ist für die Benutzung zur Anordnung auf einem Tisch oder dergleichen ausgelegt, wobei die in Figur 2 gezeigte Aufsicht gegenüber der Tischoberfläche pultförmig geneigt ist.

Das Modul 1a ist ein zentral angeordnetes Hauptmodul, welches typischerweise die meisten Bestandteile des Hörsystemprogrammiergeräts 1 und bei einer typischen Realisierung insbesondere den Computer aufnimmt. Die beiden links bzw. rechts vom Hauptmodul 1a angeordneten Seitenmodule 1b, 1c nehmen insbesondere die beiden Lautsprecher 121a, 121b auf, können aber auch weitere Komponenten, z. B. die den Lautsprechern vorgeschalteten Verstärker 120a, 120b aufnehmen. Der Abstand zwischen den Lautsprechern 121a, 121b ist so bemessen, dass für einen sich in einem typischen Bedienabstand von z. B. 30cm bis 50cm eine gute Stereoortung gegeben ist. Figur 2 zeigt das Hörsystemprogrammiergerät 1 in seinem Betriebszustand. Im Interesse kompakter Abmessungen bei Aufbewahrung und Transport sind die Seitenmodule 1b, 1c vorzugsweise über (nicht separat referenzierte) Scharniere am Hauptmodul 1a montiert, so dass sie für Transport und Aufbewahrung entlang der Scharnierlinien A, A' auf das Hauptmodul geklappt werden können. Im geschlossenen Zustand werden die Seitenmodule 1b, 1c vorzugsweise n ihrer Schliesslage gehalten, wofür vorteilhaft z. B. innenseitig liegende Permanentmagnete vorhanden sein können.

Die als Touchscreen ausgeführte Eingabe- und Anzeigeeinheit 13 nimmt einen Grossteil der Oberfläche des Hauptmoduls 1a ein. Neben der Funktion als Eingabeeinheit dient der Touchscreen 13, ggf. in Verbindung mit den Lautsprechern 121a, 121b, als allgemeine Benutzerschnittstelle zur Bedienung des Hörsystemprogrammiergeräts 1. Neben dem Touchscreen 13 kann das Hörsystemprogrammiergerät 1 bedarfsweise weitere Bedienelemente aufweisen. In Figur 2 ist beispielhaft ein Hauptschalter 13a gezeigt.

Nachfolgend wird zusätzlich auf Figur 3 Bezug genommen. Figur 3 zeigt schematisch einen hierarchischen Ansatz für eine beispielhaft Durchführung eines Verfahrens zur anwenderspezifischen Programmierung eines Hörsystems.

Die nachfolgenden Darstellungen gehen davon aus, dass ein Hörsystem 2 zur Durchführung eines erfindungsgemässen Verfahrens für einen spezifischen Anwender konfiguriert bzw. programmiert ist. Hierzu werden - in grundsätzlich bekannter Weise - audiologische Tests z. B. durch einen Hörgeräteakustiker und/oder Arzt durchgeführt und so die individuellen Gehördefizite festgestellt, aus denen sich der individuelle Verfahrensablauf bestimmt. Zur Konfiguration für einen individuellen Anwender kann das Hörsystemprogrammiergerät 2 eine Vielzahl von Modulen mit Aufgaben, Übungen und Lektionen speichern, welche in einer initialen Phase ausgewählt und zu einem individuellen Ablauf zusammengestellt werden. Die durch die zentrale Steuereinheit 10 gesteuerte und koordinierte Durchführung des Verfahrens erfolgt dann auf Grundlage dieser individuellen Programmierung bzw. Konfiguration. Alternativ kann die individuelle Konfiguration auch von einer externen Einheit wie zuvor beschrieben abgerufen bzw. übermittelt werden.

Die Durchführung des Verfahrens geht einher mit dem Absolvieren einer Reihe von Lektionen L durch den Anwender, wobei die einzelnen Lektionen L in einem zeitlichen Abstand absolviert werden. Die zu ein- und derselben Lektion L gehörenden Schritte werden im Wesentlichen unmittelbar hinter einander und in einer Einheit ausgeführt.

Jede Lektion L umfasst eine Reihe von hintereinander zu absolvierenden Übungen Ü. Jede Übung Ü adressiert einen bestimmten Themenkomplex, der bei der anwenderspezifischen Programmierung zu berücksichtigen ist.

Jede Übung Ü umfasst eine Reihe von Aufgaben A. Eine Aufgabe A umfasst die Präsentation mindestens eines Schallereignisses in Form der Abstrahlung mindestens eines Testsignals durch das Hörsystemprogrammiergerät z, auf welches der Anwender durch Eingabe einer Rückmeldung entsprechend einer vorgegebenen und z. B. über den Touchscreen 13 mitgeteilten Aufgabenstellung reagiert. Je nach Übung kann eine Übung neben einem akustischen Testsignal auch weitere Bestandteile umfassen, insbesondere eine, z. B. auf dem Touchscreen 13 dargestellte optische Information, beispielsweise ein Bild, das mit dem akustischen Testsignal in einem Sinnzusammenhang steht.

Eine Übung Ü besteht aus einer Abfolge von im Grundsatz gleichartigen Aufgaben A in unterschiedlicher spezifischer Ausprägung, wie weiter unten dargestellt. Jede absolvierte Aufgabe A lässt sich anhand der vom Anwender gegebenen Rückmeldung als alternativ "korrekt gelöst" oder "falsch gelöst" klassifizieren oder es lässt sich ein Grad beziehungsweise prozentualer Anteil ermitteln, zu dem die jeweilige Aufgabe korrekt gelöst wurde. Gleiches gilt für Übungen Ü und Lektionen L als übergeordnete Einheiten. Eine Übungsaufgabe ist dann korrekt gelöst, wenn die vom Anwender erhaltene Rückmeldung der Referenz-Rückmeldung entspricht.

Die Inhalte der einzelnen Aufgaben A, Übungen Ü und Lektionen L ist dabei derart gestaltet, dass die jeweils verwendeten Testsignale in Verbindung mit der Auswertung der Rückmeldungen des Anwenders eine Datenbasis für die anwenderspezifische Modifikation der Programmierung des Hörsystems 2 bilden. Ferner dienen sie bevorzugt einem Training des Anwenders in der zuvor dargestellten Weise, wodurch das Hörsystemprogrammiergerät 1 zugleich als Hörtrainingsgerät dient. Der hier dargestellte Ablauf mit einer Gliederung nach Aufgaben, Übungen und Lektionen stellt lediglich ein Beispiel für eine mögliche Gliederung des zeitlichen Ablaufs des Verfahrens dar.

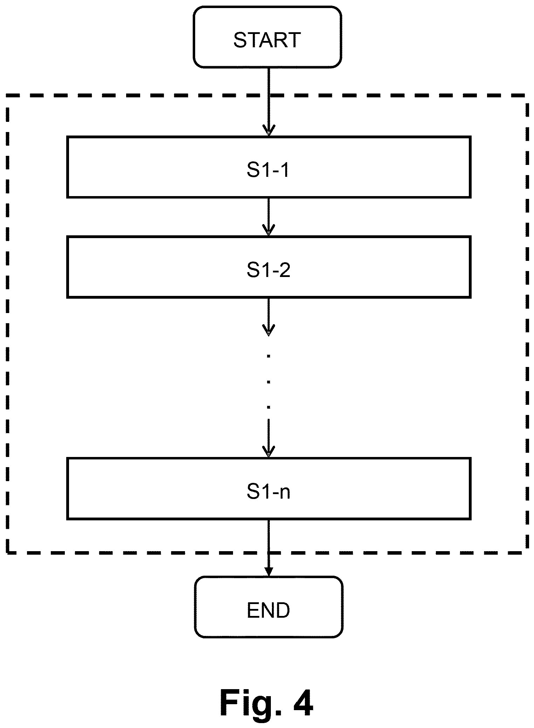

Nachfolgend wird zusätzlich auf Figur 4 Bezug genommen. Figur 4 zeigt in Form eines vereinfachten Flussdiagramms einen beispielhaften Ablauf eines Verfahrens zur anwenderspezifischen Programmierung des Hörsystems 2 mittels des Hörsystemprogrammiergeräts 1.

Die anwenderspezifische Programmierung entsprechend der vorliegenden Offenbarung erfolgt in Schritten S1-1 bis S1-n, welche jeweils in grundsätzlich gleicher Weise ablaufen und jeweils die Durchführung einer Lektion L umfassen. Die anwenderspezifische Programmierung wird zeitlich über n Tage - beispielsweise aufeinanderfolgende Tage - durchgeführt, wobei an jedem Tag x ein entsprechender Schritt S1-x ausgeführt wird. Das Ausführen des Schrittes S-1-x geht einher mit dem Absolvieren einer entsprechenden Lektion L-x. Die Gesamtdauer des Verfahrens kann beispielsweise 20 oder 30 Tage betragen, entsprechend n=20 bzw. n=30. Die Dauer für die Durchführung jedes Schrittes S1-x und damit die Dauer zum Absolvieren einer Lektion durch den Anwender beträgt beispielsweise ungefähr 45 min. Die einzelnen Lektionen sind aufeinander aufbauend angelegt und besitzen eine mit der Zeit fortschreitende Komplexität und Schwierigkeit, wie weiter unten näher beschrieben. Dabei ist jeder der Schritte S1-1 bis S1-m grundsätzlich gleich aufgebaut, wie nachfolgend beschrieben.

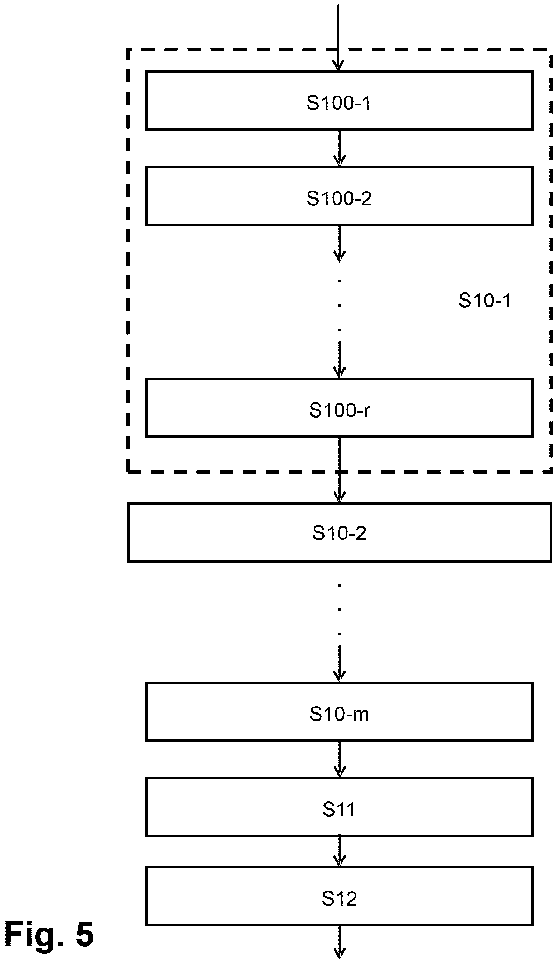

Nachfolgend wird zusätzlich auf Figur 5 Bezug genommen. Figur 5 zeigt in Form eines vereinfachten Flussdiagramms einen beispielhaften Ablauf für einen einzelnen Schritt S1-x nach Figur 4.

Die dem Schritt S1-x zugeordnete Lektion L-x umfasst das Absolvieren einer Reihe von Übungen, denen die aufeinanderfolgenden Schritte S10-1 bis S10-m zugeordnet sind, wobei m die Zahl der Übungen einer Lektion bezeichnet. Die Zahl m der Übungen kann dabei für die verschiedenen Lektionen L-x gleich oder unterschiedlich sein. Typischerweise liegt m beispielsweise in einem Bereich von drei bis acht.

Jede Übung umfasst das Absolvieren einer Reihe von Aufgaben, denen die aufeinanderfolgenden Verfahrensschritte S100-x-1 bis S100-x-r zugeordnet sind. Dabei ist jeder der Schritte S10-1 bis S10-m grundsätzlich so aufgebaut, wie in Figur 5 für den Schritt S-10-1 beispielhaft dargestellt. Die Zahl r der Aufgaben kann dabei für die einzelnen Übungen gleich oder unterschiedlich sein.

Optional wird nach Abschluss jeder Aufgabe oder ggf. Übung eine Anzeige an den Anwender gegeben, ob bzw. in welchem Umfang die Aufgabe/Übung korrekt gelöst wurde. Dies kann beispielsweise durch textuelle und/oder symbolische und/oder farbliche Anzeigen auf dem Touchscreen 13 erfolgen. Ferner kann die optionale Möglichkeit vorgesehen werden, eine "falsch" gelöste Aufgabe zu wiederholen, wobei die Gesamtzahl der Wiederholungen auf eine Anzahl von beispielsweise drei begrenzt seien kann.

Im Anschluss an das Absolvieren der letzten Übung m, erfolgt im Schritt S11-x eine Auswertung, in der die Programm-Modifikationseinheit 14 und/oder die zentrale Steuereinheit 10 eine Auswertung der in den einzelnen Übungen und Aufgaben über den Touchscreen 13 empfangenen Rückmeldungen vornimmt. Basierend auf dieser Auswertung wird, wie nachfolgend beschrieben, durch die Programm-Modifikationseinheit 14 eine modifizierte Programmierung für das Hörsystem 2 ermittelt und im lernfähigen Konfigurationsspeicher 16 gespeichert.

Während in Figur 5 die Bestimmung der modifizierten Programmierung als separater Schritt S11 dargestellt ist, kann die Durchführung des Schrittes S11 auch zeitlich verteilt erfolgen, beispielsweise jeweils unmittelbar im Anschluss an das Beenden einer Übung und damit jeweils innerhalb der Schritte S10-1, S10-2 ... S10-m. Ebenso kann es kontinuierlich während dem Absolvieren der Aufgaben und/order Übungen erfolgen.

Im weiter nachfolgenden Schritt S12 erfolgt, mittels der Kommunikationseinheit 15 des Hörsystemprogrammiergeräts 1 und der Kommunikationseinheit 21 des Hörsystems 2, eine Übertragung der in Schritt S12 ermittelten modifizierten Programmierung und eine Änderung der Programmierung des Hörsystems 2 von der zu Beginn des Schritts S1-x gegebenen initialen Programmierung zu einer demgegenüber modifizierten Programmierung. Diese modifizierte Programmierung bildet wiederum die initiale Programmierung für die nächste nachfolgende Lektion x+1 und damit den nachfolgenden Schritt S1-[x+1]. Alternativ besteht, ebenso wie für den Schritt S11 zuvor beschrieben, ferner die Möglichkeit, die Änderung der Programmierung und damit die Ausführung des Schrittes S12 zeitlich verteilt vorzunehmen.

Der lernfähige Konfigurationsspeicher 16 speichert die sich während der Anwendung des Verfahrens ändernde Programmierung des Hörsystems 2 ab und stellt somit ein entsprechend der Modifikation der Programmierung des Hörsystems 2 adaptiven Speicher für die Programmierung des Hörsystems 2 dar.

In weiteren alternativen Varianten wird der Schritt S12 und gegebenenfalls auch der Schritt S11 nicht innerhalb jedes Schrittes S1-x und damit im Rahmen der Absolvierung jeder Lektion ausgeführt, sondern nur innerhalb eines Teiles dieser Schritte, zum Beispiel im Rahmen jedes zweiten, dritten, oder fünften Schrittes S1-x.

In der hier beschriebenen beispielhaften Ausführungsform erfolgt die kleinste und damit elementare Form einer Datensammelsequenz durch jede Aufgabe A. Jede Aufgabe A ist auf einen Aspekt oder eine Anzahl spezifischer Aspekte des Hörens und der akustischen Wahrnehmung ausgerichtet, die ein Mensch üblicherweise zur Wahrnehmung und Verarbeitung akustischer Signale beherrscht und deren Beherrschung durch einen mit einer Hörhilfe ausgestatteten Anwender massgeblich für den im Alltag aus dem Einsatz der Hörhilfe erzielten Nutzen ist. Einige Beispiele derartiger Aspekte (auch "Disziplinen") genannt: sind (beispielhaft): Sprachverstehen; dichotisches Hören; Verknüpfung von akustischer und optischer Wahrnehmung; Assoziatives Hören. Nachfolgende Tabelle gibt beispielhaft für verschiedene Disziplinen beispielhafte Übungstypen an.

| Disziplin | Aufgabe(n) /Übung(n) |
|---|---|
| Sprachverstehen | verstehen zweistelliger und dreistelliger oder allgemein x-stelliger Zahlenwörter; |
| | Verstehen einzelner Wörter verschiedener Silbenzahl, insbesondere "ähnlich" klingender Wörter (z. B. Dinkel, Dunkel, Winkel); |
| | Verstehen einzelner Silben; |
| | Verstehen von Sätzen und Fliesstext; |
| | Verstehen sowohl vorwärts als in veränderter Reihenfolge (z. B. rückwärts) wiedergegebener Zahlenwörter, Ziffernfolgen oder Wortfolgen. |
| Dichotisches Hören | Verstehen des von einem Kanal wiedergegebenen Inhalts (z. B einen gesprochenen Satz) eines Kanals, während gleichzeitig über den anderen Kanal ein anderer Inhalt (anderer gesprochener Satz) wiedergegeben wird. |
| Verknüpfung akustischer und optischer Wahrnehmung | Zuordnung eines gehörten akustischen Reizes bzw. einer akustischen Wahrnehmung (z. B. eines Begriffes; Satzes; Klangs; Geräusches; einer Melodie) zu einem von mehreren auf dem Touchscreen dargestellten Bildern (optischer Reiz; optische Wahrnehmung) |
| Assoziatives Hören | Zählen der Häufigkeit von Zahlen, Wörtern, oder charakteristischen Klängen (z. B. Klirren von Glas) in einer längeren gehörten Passage. |

Für die einzelnen Aufgaben /Übungen wird jeweils die Benutzerschnittstelle des Hörsystemprogrammiergeräts genutzt, z. B. der Touchscreen 13, welcher jeweils eine der Übung entsprechende graphische Benutzerschnittstelle bereitstellt, z. B. eine alphanumerische Tastatur, Auswahlboxen, Spin-Wheels, etc.

Für einen Teil der Disziplinen mit den jeweils zugehörigen Aufgaben /Übungen, zum Beispiel das Sprachverstehen, wird über beide Wiedergabekanäle des Hörsystemprogrammiergeräts 1 ein jeweils identisches Testsignale abgegeben. Bei anderen Disziplinen, insbesondere dem dichotischen Hören und der Stereo-Lokalisation, unterscheidet sich dagegen das Testsignale für den linken und den rechten Kanal.

Die vom Anwender zu verstehenden Inhalte (Wörter, Silben, Zahlen, Sätze, usw.) stellen jeweils ein Nutzsignal dar. Dem Nutzsignal kann ein Störsignal überlagert werden. Das Störsignal ist hier erfindungsgemäss ein im Wesentlichen gleichförmiges Störsignal und/oder ein kurzzeitiges Impulssignal. Beispiele für gleichförmige Störsignale sind allgemeiner Verkehrslärm, die typische Geräuschkulisse eines Restaurants oder Grossraumbüros, das Rauschen eines Baches, Brandungsrauschen etc. Beispiele für impulsförmige Störsignale sind eine Kirchenglocke, und kurzes Hundegebell, oder Telefonklingeln.

Die zentrale Steuereinheit 10 steuert die Zusammensetzung der Testsignale aus Nutzsignal und Störsignal so, dass mit zunehmender Zeit der Störanteil bzw. Störpegel grösser wird, d.h. das Signal-Rausch-Verhältnis (SNR) abnimmt. Bei der beispielhaften Einteilung des Verfahrens gemäss Figur 4 kann der Störpegel zum Beispiel mit jedem der Schritte S1-x steigen, oder nur mit jedem zum Beispiel zweiten oder dritten Schritt. Bei einer Steigerung des Störpegels zwischen jedem Schritt S1-x und dem nachfolgenden Schritt S1-[x+1] sinkt der Signal-Rausch-Abstand demnach jeweils von Schritt zu Schritt. Bei der beispielhaften Durchführung eines Schrittes S1-x pro Tag wird dementsprechend der Signal-Rausch-Abstand von Tag zu Tag etwas geringer. In Abhängigkeit der vom Anmelder über die Eingabeeinheit (13) abgegebenen Rückmeldungen kann der Störpegel durch die zentrale Steuereinheit (10) aber auch für verschiedene Tage gleichbleiben oder auch verringert werden, wie weiter unten näher ausgeführt.

Neben einer grundsätzlichen Zunahme des Störpegels über die Zeit, wie beschrieben, steuert die zentrale Steuereinheit 10 den Störpegel ferner in Abhängigkeit der vom Anwender beim Absolvieren der einzelnen Aufgaben /Übungen empfangenen Rückmeldungen. Hierbei wird insbesondere ausgewertet, zu welchem Anteil die jeweiligen Übungen vom Anwender korrekt gelöst wurden. Je besser die Übungen und Aufgaben vom Anwender gelöst worden, d.h. je grösser der Anteil der einer korrekten Antwort entsprechenden Rückmeldungen ist (in anderen Worten, je grösser der Anteil der der jeweiligen Referenz-Rückmeldung entsprechenden Rückmeldungen ist), umso stärker wird der Störpegel erhöht bzw. das Signal-Rausch-Verhältnis herabgesetzt. Hierdurch wird erreicht, dass dem Anwender während der Anwendung des Verfahrens zunehmend schwierigere Aufgaben /Übungen präsentiert, der Anwender aber unter den jeweils gegebenen konkreten Umständen auch nicht überfordert wird. Die hier beschriebene Steuerung des Störpegels in Abhängigkeit der Rückmeldungen des Anwenders ist insbesondere unter dem Aspekt des Gehörtrainings vorteilhaft, hinsichtlich der Modifikation der Programmierung des Hörsystems aber nicht zwingend.

Die zeitgesteuerte Erhöhung des Störpegels kann zum Beispiel zwischen den einzelnen Schritten S1-x jeweils gleichmässig erfolgen, wobei beispielsweise der Signal-Rausch-Abstand jeweils um gleiche Pegel gesenkt wird. Da ferner die Ausblendung impulsförmiger Störsignale für den Anwender schwieriger ist als die Ausblendung im Wesentlichen kontinuierlicher Signale, kann ferner vorgesehen werden, impulsförmige Störsignale erst in einem fortgeschrittenen Stand der Anwendung des Verfahrens vorzusehen oder, je nach spezifischer Situation, auch vollständig zu unterlassen.

ist der Anteil von Rückmeldungen, die einer jeweils korrekten Antwort entsprechend gering, kann der Störpegel auch unverändert gelassen oder auch gesenkt werden. Hierfür kann die zentrale Steuereinheit 10 die Anzahl oder den Anteil jeweils korrekt gelöster Aufgaben mit einstellbaren oder fix vorgegebenen Schwellwerten vergleichen und den Störpegel in Abhängigkeit des Vergleiches anpassen. So kann jeweils ein unterer und ein oberer Schwellwert für die Zahl bzw. den Anteil korrekter Antworten gespeichert sein. Bei Unterschreiten des unteren Schwellwertes reduziert die zentrale Steuereinheit den Störpegel, bei überschreiten des oberen Schwellwertes erhöht sie ihn entsprechend. Liegt die Zahl bzw. der Anteil der korrekten Antworten zwischen den Schwellwerten, bleibt der Störpegel unverändert. Entsprechende Schwellwerte können für alle Übungen identisch oder für zumindest einige Übungen unterschiedlich sein.

Für besondere Fälle, beispielsweise einen bereits weit fortgeschrittenen Hörverlust, bei Anwendern bzw. Patienten mit weiteren relevanten Erkrankungen, beispielsweise Alzheimer-Syndrom, etc. ist es auch möglich, auf das Störsignal ganz oder teilweise zu verzichten, so dass die Testsignale lediglich ein Nutzsignal umfassen. Ein solcher Verzicht kann ebenfalls angezeigt sein im Zuge der Verbesserung der Unterscheidbarkeit ähnlich klingender Laute, wie weiter unten näher beschrieben.

Die Auswahl des Nutzsignals in den einzelnen Übungen kann teilweise zufallsgesteuert erfolgen. So können etwa Zahlen, Wörter, Silben und Sätze durch die zentrale Steuereinheit 10 und/oder den Testsignalgenerator 11, insbesondere den Nutzsignalgenerator 11a mittels eines Zufallsgenerators aus einer umfangreicheren Sammlung von Zahlen, Buchstaben, Wörtern, Sätzen usw., welche im Testsignalgenerator 11 gespeichert sind, ausgewählt werden. In ähnlicher Weise kann die Auswahl von Störsignalen ganz oder teilweise zufallsgesteuert erfolgen.

Die Durchführung des Verfahrens in der zuvor beschriebenen Weise in zum Beispiel täglichen Lektionen erfolgt vorzugsweise derart, dass die grundsätzliche Komplexität und Schwierigkeit der jeweiligen Aufgaben, ebenso wie der Störpegel gemäss obiger Darstellung, über die Zeit zunimmt. Weitere Erschwernisse können z. B. durch Massnahmen wie eine Erhöhung der Sprechgeschwindigkeit und/oder ein Wechsel zwischen verschiedenen Sprechern mit verschiedenen Stimmen und/oder verschiedenen Lautstärken erreicht werden.

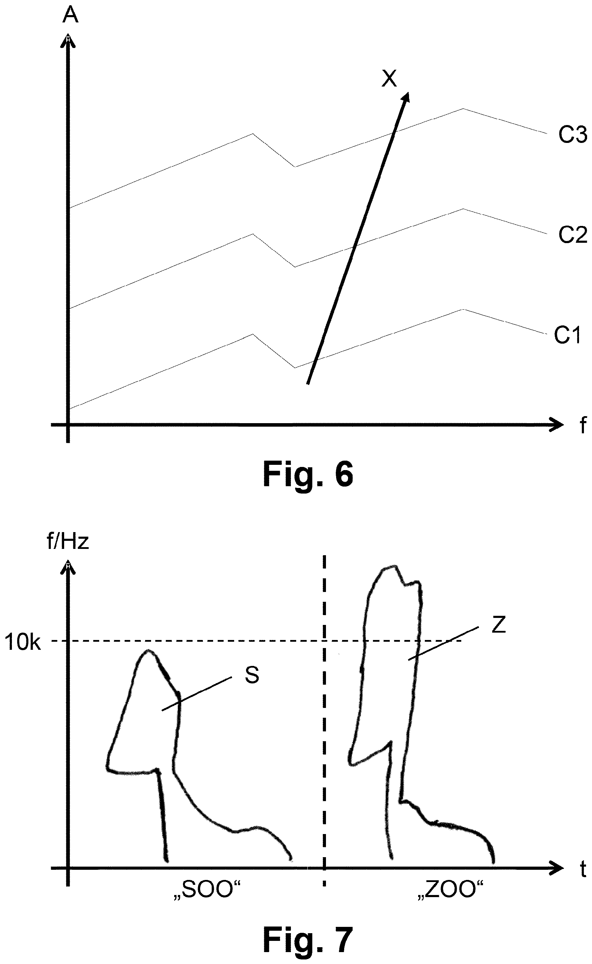

Wie zuvor erwähnt, erfolgt in den Schritten S11, S12 jeweils eine Modifikation der Programmierung des Hörsystems 2. Zur näheren Erläuterung dieses Aspekts wird nachfolgend zusätzlich auf Figur 6 Bezug genommen, die in einen ersten Aspekt einer Änderung der Programmierung des Hörsystems 2 durch das Hörsystemprogrammiergerät 1 in den Schritten S11, S12 veranschaulicht, insbesondere die Bestimmung einer frequenzunabhängig modifizierten Verstärkung.

Das Hörsystem 2 weist eine in Abhängigkeit der Frequenz f programmierbare Verstärkung A auf, wie grundsätzlich aus dem Stand der Technik bekannt. Die Verstärkung A lässt sich in einem Diagramm als Kurve über die Frequenz darstellen. Figur 6 zeigt beispielhaft drei als stückweise linear angenommenen Kurven C1, C2, C3, wobei die Ordinate des Diagramms entsprechend einer Pegeldarstellung logarithmisch ist. Der qualitative Verlauf der Kurven entspricht dabei der (frequenzabhängigen) Verstärkung, die erforderlich ist, um den ohne Einsatz des Hörsystems 2 bestehenden Verlust an Hörvermögen zu kompensieren.

Für einen idealerweise vollständigen Ausgleich des Verlusts an Hörvermögen wäre es grundsätzlich wünschenswert, die Verstärkung A so einzustellen, dass die vom geschädigten Gehör mit Hörsystem wahrgenommene Lautstärke der vom unbeschädigten Gehör ohne Hörhilfe wahrgenommen Lautstärke entspricht. In der Praxis ist dies jedoch kaum vollständig möglich. Dies liegt insbesondere daran, dass bei bestehendem Hörverlust das Gehirn rasch die Fähigkeit verliert, Störgeräusche in der Wahrnehmung zu unterdrücken und damit auszublenden bzw. zu maskieren. Ein vollständiger oder zumindest weitgehender Ausgleich des Hörverlustes durch entsprechend hohe Einstellung der Verstärkung würde daher zu einer als subjektiv unerträglich laut empfundenen und gegebenenfalls auch schmerzhaften Wahrnehmung von Störgeräuschen führen.

Entsprechend einigen Ausführungsformen der vorliegenden Erfindung wird die Verstärkung des Hörsystems über einen längeren Zeitraum verteilt und zum Beispiel schrittweise vergrössert. Hierdurch wird erreicht, dass das Gehör des Anwenders über einen längeren Zeitraum an höhere Verstärkungen gewöhnt wird, ohne dass die über die Zeit zunehmende zunehmenden Verstärkung zu einer unakzeptablen Zunahme der subjektiv wahrgenommenen Lautstärke der Störgeräusche führt. Entsprechend wird die Verstärkung A, wie durch den Pfeil X angedeutet, über die Zeit von der beispielhaften initiale Kurve C1, über die Kurve C2 als beispielhaften Zwischenstand bis zur Kurve C3 als beispielhaften Endzustand bei Abschluss des Verfahrens erhöht, wobei die Kurve C3 die Zielverstärkung in Funktion der Frequenz darstellt.

Eine frequenzunabhängige Änderung der Verstärkung der zuvor dargestellten Art kann zeitgesteuert und unabhängig von den vom Anwender in Reaktion auf die Testsignale erhaltenen Rückmeldungen sein. Optional können jedoch Rückmeldungen des Anwenders berücksichtig werden. Diese Rückmeldungen sind typischerweise keine Antworten auf Aufgaben der zuvor dargestellten Art und unterliegen typischerweise nicht der zuvor dargestellten binären Einteilung aufgrund des Vergleichs mit einer Referenz-Rückmeldung. Derartige Rückmeldungen betreffen vielmehr das Wohlbefinden und den Hörkomfort des Anwenders. So kann insbesondere bei einer Durchführung eines erfindungsgemässen Verfahrens über einen längeren Zeitraum von mehreren Wochen zu verschiedenen Zeitpunkten, z. B. im Rahmen der jedes Schrittes der schrittweisen Erhöhung der Verstärkung vorgesehen werden, dass der Anwender jeweils angibt, ob ihm die aktuelle Verstärkung angenehm, grenzwertig laut oder zu laut (unerträglich laut) ist. In Abhängigkeit der Rückmeldung kann das Verfahren die vorgesehene Zeit bis zur Erreichung des Endzustandes (Kurve C3) zeitlich strecken, die angestrebte Verstärkung im Zielzustand verringern oder auch - im Falle einer akut zu hohen Verstärkung - die aktuelle Verstärkung reduzieren. Ferner kann vorgesehen werden, dass der Anwender den Zeitpunkt des nächsten Schritts einer Erhöhung der Verstärkung in Richtung des angestrebten Endzustands durch eine Eingabe selbst auslöst.

Alternativ oder zusätzlich zur Änderung der Programmierung des Hörsystems durch eine allgemeine frequenzunabhängige (unspezifische) Änderung der Verstärkung wie zuvor beschrieben kann das Verfahren eine selektive Änderung der Verstärkung bei spezifischen Frequenzen beziehungsweise in spezifischen Frequenzbereichen beinhalten. Mit dieser Massnahme kann das Differenzierungsvermögen bezüglich ähnlich klingender Buchstaben, Silben oder Phoneme (hier zusammenfassend als "Laute" bezeichnet) gezielt verbessert werden.

Nachfolgend wird zusätzlich auf Figur 7 Bezug genommen. Figur 7 zeigt beispielhaft und in schematischer Weise in einem gemeinsamen Diagramm die wesentlichen Umrisse der Spektrogramme für ein gesprochenes "SOO" (links) bzw. ein gesprochenes und "ZOO" (rechts). Es ist ersichtlich, dass sich die Spektrogramme insbesondere in einem Frequenzbereich oberhalb 6kHz unterscheiden. Dieser Frequenzbereich ist bei "ZOO" deutlich vorhanden, bei "SOO" dagegen nicht.

Entsprechend der vorliegenden Erfindung wird für einen Anwender, welcher die den gesprochenen "S" und "Z" entsprechenden Laute einzeln und/oder in Wörtern nicht oder nur unzureichend zu differenzieren vermag, die Programmierung des Hörsystems 2 in der Weise verändert, dass die Verstärkung in diesem differenzierenden Frequenzbereich selektiv angehoben wird. Hierdurch erhöht sich der wahrgenommene Unterschied und damit das Differenzierungsvermögen zwischen den Lauten.

Im Beispiel der Figur 7 erfolgt die Differenzierung zwischen "S" und "Z" über einen einzelnen engen Frequenzbereich. Die Differenzierung kann aber bei anderen Lauten bzw. Lautpaaren auch über zwei oder mehr Frequenzen bzw. Frequenzbereiche erfolgen, die entweder lediglich im Spektrum eines der Laute vorkommen oder aber in den Spektren beider Laute vorhanden sind, aber mit deutlich unterschiedlichem Amplituden- bzw. Leistungspegel. Derartige Frequenzen und Frequenzbereiche, die zur Differenzierung ähnlich klingender Laute herangezogen werden können, werden hier summarisch als differenzierende Frequenzbereiche bezeichnet.

Im Nutzsignalgenerator 11a des Testsignalgenerators 11 sind hierfür Nutzsignale mit schwierig differenzierbaren Lauten als gesprochene Buchstaben, Silben, Wörter, und/oder Phoneme hinterlegt. In der Programm-Modifikationseinheit 14 können in diesen Ausführungsformen die entsprechenden spektralen Bestandteile abgespeichert sein, zum Beispiel in Form tabellarisch abgelegter Spektrogramme gemäss Figur 7. Alternativ oder zusätzlich können für Paare und/oder Gruppen von Lauten auch unmittelbar die differenzierenden Frequenzen bzw. Frequenzbereich gespeichert sein. Die Spektrogramme einzelner gesprochener Laute sind in ihrem qualitativen Verlauf weitgehend invariant bezüglich der sprechenden Person, so dass sie oder aus ihnen abgeleitete Grössen in allgemeiner Form gespeichert werden können. Gestalt der Spektrogramme für verschiedene Laute sowie Verfahren zu ihrer Bestimmung sind im technischen Gebiet der Hörakustik grundsätzlich bekannt.

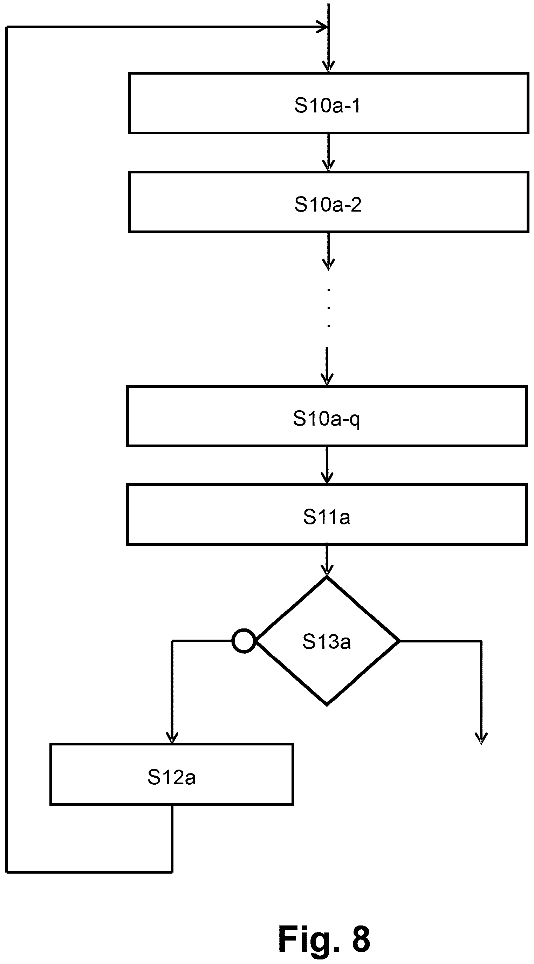

Ein Ablauf beispielhafter Verfahrensschritte zur frequenzabhängigen Änderung der Programmierung nach dem zuvor dargestellten Ansatz wird nachfolgend mit zusätzlichem Bezug auf Figur 8 dargestellt. In einer Datensammelsequenz mit den Schritten S10a-1 bis S10a-m werden Daten zum Differenzierungsvermögen hinsichtlich schwierig differenzierbarer Laute erhoben. Die einzelnen Übungen mit den Schritten S10a-x werden dabei in der zuvor beschriebenen Weise durchgeführt. Es wird hier angenommen, dass lediglich das Differenzierungsvermögen zwischen zwei Lauten, zum Beispiel "S" und "Z" festgestellt und erforderlichenfalls verbessert werden soll. Für verschiedene Laute bzw. Lautgruppen kann das Verfahren wie hier beschrieben sequenziell durchgeführt werden. Grundsätzlich ist es jedoch auch möglich, das Verfahren gleichzeitig hinsichtlich einer Reihe von Lauten bzw. Lautpaaren durchzuführen und nachfolgend die Programmierung des Hörsystems 2 in einem gemeinsamen Schritt zu modifizieren. Ferner wird in der Darstellung von Figur 8 davon ausgegangen, dass die Datensammlung in Form einer Reihe unterschiedlicher Übungen erfolgt, denen die Schritte S10a-1 bis S10a-q zugeordnet sind und jede der Übungen eine Abfolge von Aufgaben umfasst. Die einzelnen Aufgaben können zum Beispiel getrennt auf die Unterscheidbarkeit einzelner Buchstaben, Silben, und Worte gerichtet sein. Selbstverständlich ist aber es auch möglich, lediglich eine Übung mit einer entsprechenden Anzahl gleichartiger Aufgaben vorzusehen.

Im Anschluss an die Aufgaben/Übungen wird in Schritt S11a eine Auswertung in der zuvor beschriebenen Weise vorgenommen. Dadurch wird ermittelt, in wieweit bzw. zu welchem Anteil die Aufgaben /Übungen vom Anwender korrekt gelöst worden. Im anschliessenden Schritt S13a wird festgestellt, ob das Differenzierungsvermögen des Anwenders zufriedenstellend bzw. ausreichend ist. Dies kann beispielsweise durch einen Vergleich des Anteils korrekt gelöst war Aufgaben /Übungen mit einem Schwellenwert erfolgen. Im positiven Falle ist keine Modifikation der Programmierung des Hörsystems 2 erforderlich und das Verfahren wird mit weiteren Aufgaben, Übungen oder Lektionen fortgesetzt. Im negativen Fall erfolgt im Schritt S12a die Bestimmung einer modifizierten Programmierung des Hörsystems 2 durch die Programm-Modifikationseinheit und eine entsprechende Änderung der Programmierung. Dafür wird, wie mit Bezug auf Figur 7 zuvor beschrieben, die Verstärkung für einen differenzierenden Frequenzbereich oder mehrere differenzierende Frequenzbereiche selektiv modifiziert. Neben einer frequenzselektiven Erhöhung ist dabei auch eine frequenzselektive Absenkung der Verstärkung möglich.

Im Anschluss an die Modifikation der Programmierung des Hörsystems 2 erfolgt vorzugsweise die Durchführung einer erneuten Datensammlungssequenz und einer entsprechenden Auswertung sowie die Beurteilung hinsichtlich zufriedenstellender beziehungsweise ausreichendem Differenzierungsvermögen. Hierdurch kann festgestellt werden, ob die angestrebte Verbesserung des Differenzierungsvermögens erreicht worden ist. Falls dies nicht der Fall ist, kann Schritt S12a erneut durchgeführt und damit die Verstärkung weiter modifiziert werden.

Grundsätzlich kann Schritt S12a und damit die frequenzabhängige Modifikation der Verstärkung vielfach hintereinander durchgeführt werden. Tatsächlich sind der Anhebung der Verstärkung dadurch Grenzen gesetzt, dass im differenzierenden betreffenden Frequenzbereich auch Störsignale entsprechend verstärkt werden. Daher kann die Anhebung der Verstärkung im Schritt S12a aus Sicherheitsgründen auf einen vorgegebenen Maximalwert beschränkt werden.

Im Schritt S12a kann die Verstärkung jeweils um einen festen Betrag geändert, z. B. angehoben werden. Alternativ kann die Änderung jedoch auch in Abhängigkeit der vorangehenden Auswertung im Schritt S13a vorgenommen werden. Weist etwa der Anwender hinsichtlich der Differenzierung zweier Laute ein erhebliches Defizit auf (gekennzeichnet durch einen geringen Teil korrekter Lösungen der Aufgaben/Übungen), kann im Schritt S12a eine relativ grosse Änderung der Verstärkung eines oder mehrerer spezifischer Frequenzbereiche erfolgen. Bei einem zwar vorhandenen, aber geringeren Defizite hinsichtlich der Differenzierung der Laute kann im Schritt S12a dagegen eine geringere Änderung der Verstärkung erfolgen.

In den Datensammelschritten S10a-1 bis S10a-q werden die Nutzsignale in einer beispielhaften Ausgestaltung zunächst nicht von einem Störsignal überlagert. Im Anschluss an die gegebenenfalls erfolgte Änderung der Programmierung kann in nachfolgenden (in Figur 8 nicht explizit dargestellten) Schritten der Pegel des Störsignals erhöht werden. Ebenfalls ist es aber auch möglich, in der Datensammelsequenz mit den Schritten S10a-1 bis S10a-q unmittelbar eine Überlagerung von Nutzschall und Störschall vorzunehmen oder Testsignale mit verschiedenen Störpegeln vorzusehen.

Weitere Beispiele von im Alltag vorkommenden Lauten, bzw. Lautgruppen, bei denen die Differenzierung verschiedener Konsonanten häufig kritisch ist und deren Differenzierungsvermögen entsprechend durch eine Verstärkungsänderung in ausgewählten Frequenzbereichen erfindungsgemäss verbessert werden kann, sind: "AZA - ADA - AGA - ATA - ASA - ABA - AFA - AKA - ALA - ANA - APA - ACHA - AWA" oder "IFI - IZI - ISI - IGI".

Im Gegensatz zum zuvor dargestellten Beispiel der Laute "SOO" und "ZOO" ist sind die beiden letztgenannten Beispiele nicht auf genau zwei Alternativen beschränkt, sondern beziehen sich auf eine grössere Zahl jeweils ähnlich klingender Laute. Das Vorgehen kann hier dem von Figur 8 Verfahrensablauf folgen, wobei im Schritt S11a vorteilhaft festgestellt wird, zwischen welchen Lauten jeweils ein unzureichendes Differenzierungsvermögen besteht und sich die Bestimmung der Modifizierten Programmierung im Schritt S12a auf jeweils für diese Laute charakteristische Frequenzbereiche bezieht. Ebenso kann das Verfahren nach Figur 8 für verschiedene Lautgruppen separat und/oder gemeinsam durchgeführt werden. Bei einer gemeinsamen Durchführung können z. B, dem Anwender Testsignale aus verschiedenen Lautpaaren bzw. Lautgruppen in zufallsgesteuerter Reihenfolge präsentiert werden.

In einem weiteren Beispiel für die frequenzabhängige Änderung der Verstärkung gemäss Figur 8 bestehen die Testsignale nicht aus sprachlichen Lauten, Silben oder Phonemen wie zuvor dargestellt, sondern aus Klängen z. B. unterschiedlicher und ähnlich klingender Musikinstrumente, die durch den Anwender zu erkennen sind. Die frequenzabhängige Änderung der Verstärkung umfasst dann eine Änderung des bzw. der für den unterschiedlichen Klang der Instrumente charakteristischen Frequenzbereichs bzw. Frequenzbereiche (Obertonspektrum).

In einem weiteren Beispiel für die Modifikation der Verstärkung gemäss Figur 8 erfolgt die Modifikation frequenzunabhängig. Dies kann z. B. dann sinnvoll sein, wenn der Anwender ein Defizit hinsichtlich der Richtungslokalisation bzw. der Stereo-Ortung aufweist. In diesem Fall werden in den Schritten S10a-1 ... S10a-q Testsignale aus verschiedenen Richtungen abgestrahlt, z. B. "links", "mittig", "rechts", wobei auch weitere Zwischenstufen vorgesehen werden können. Die Rückmeldung des Anwenders besteht in einer Angabe der gehörten Richtung des wahrgenommenen Schallereignisses. Durch die Auswertung in den Schritten S11a/S13 wird dann festgestellt, ob - sofern der Anwender je Ohr eine Hörhilfe verwendet - die Verstärkung einer der Hörhilfen im Vergleich zur anderen zu gering ist, was sich in einem Schlechten Lokalisationsvermögen in dieser Richtung äussert. In diesem Fall wird die die Verstärkung im Schritt S12a entsprechend angepasst, z B. durch Erhöhung der Verstärkung in einem bzw. die Verringerung der Verstärkung im anderen Hörsystem.

Die erforderlichen Daten für eine frequenzabhängige und/oder frequenzunabhängige Modifikation der Verstärkung können im Hörsystemprogrammiergerät 2 z. B. in Form von Listen oder Tabellen (Look-Up-Tafeln) gespeichert sein, in welchen z. B, die für verschiedene Phoneme bzw. Phonemkombinationen charakteristisch Unterschiedlichen Frequenzbereiche (siehe Figur 7 und zugehörige obige Darstellung) hinterlegt sind. Es ist jedoch auch möglich, dass derartige Daten ganz oder teilweise in einer zentralen Einheit, wie im Zusammenhang von Figur 1 erläutert, gespeichert und vom Hörsystemprogrammiergerät 2 über eine z. B. internetbasierte Kommunikationsschnittstelle abgerufen bzw. diesem Übermittelt werden.

Sowohl eine frequenzunabhängige als auch eine frequenzabhängige Änderung der Verstärkung können grundsätzlich gemeinsam vorgenommen werden oder in einer Folge abwechselnd vorgenommen werden. In einer speziellen Ausführungsform jedoch wird das Verfahren gemäss Figur 1 in zwei aufeinanderfolgenden Programmierstufen durchgeführt. In der ersten Programmierstufe wird dabei die Verstärkung lediglich frequenzunabhängig verändert, insbesondere erhöht, wie in Figur 6 dargestellt und zuvor beschrieben. In der anschliessenden zweiten Programmierstufe wird die Verstärkung lediglich selektiv und frequenzabhängig zur Verbesserung des Differenzierungsvermögens entsprechend Figur 7 und zugehöriger Beschreibung verändert. In einer beispielhaften Ausgestaltung kann sich die Durchführung der ersten Programmierstufe z. B. über einen Zeitraum von 20 Tagen und die zweite Programmierstufe über einen nachfolgenden Zeitraum von weiteren 10 Tagen erstrecken, wobei jede der Phasen eine abfolge von Lektionen, Aufgaben und Übungen gemäss obiger Darstellung umfassen kann.

Ebenso wie bezüglich der frequenzunabhängigen Modifikation der Verstärkung des Hörsystems 2 beschrieben werden die vorgenommenen Modifikationen in der Programmierung des Hörsystems 2 im lernfähigen Konfigurationsspeicher 16 gespeichert.

In einer Variante ist es ferner möglich, den lernfähigen Konfigurationsspeicher anstatt oder zusätzlich im Hörgeräteprogrammiergerät 1 im Hörsystem 2 vorzusehen. Bei derartigen Ausführungsformen erfolgt bei bzw. nach dem Herstellen einer Kommunikationsverbindung zwischen den Kommunikationsschnittstellen 15 und 21 jeweils eine Übertragung der Programmierung vom Hörsystem 2 auf das Hörgeräteprogrammiergerät 1 bzw. ein Abgleich zwischen der im Hörsystem 2 und dem Hörgeräteprogrammiergerät 1 gespeicherten Programmierung. In Ausführungsformen, in denen im Hörsystemprogrammiergerät, wie in Figur 1 gezeigt, weiterhin der lernfähige Konfigurationsspeicher 16 vorhanden, ist, kann die Speicherung einer geänderten Programmierung zunächst lokal in diesem Konfigurationsspeicher abgespeichert werden und von diesem anschliessend an das Hörsystem 2 übertragen werden. In Ausführungsformen, in denen ein lernfähiger Konfigurationsspeicher nur im Hörsystem vorgesehen ist, kann die Speicherung der geänderten Programmierung unmittelbar dort erfolgen.

Derzeitige Hörsysteme mit digitalen Signalprozessor entsprechend dem Stand der Technik erlauben es ferner, die Verstärkung in Funktion der Frequenz (siehe Figur 6) für unterschiedliche Eingangslautstärken des auf das Mikrofon bzw. die Mikrofone des Hörsystems treffende Geräusche unterschiedlich zu programmieren, d. h. verschieden laute Geräusche unterschiedlich in Abhängigkeit der Frequenz unterschiedlich stark zu verstärken, wobei typischerweise einen Einteilung in z. B. vier Bereiche von (Eingangs-)Lautstärken erfolgt. Ebenso können Hördefizite sich ausschliesslich oder vornehmlich auf bestimmte Lautstärkebereiche beziehen. Erfindungsgemässe Verfahren und Hörsystemprogrammiergeräte können dementsprechend derart gestaltet sein, dass die die zuvor beschriebenen Verfahren ganz oder teilweise separat für verschiedene (Eingangs-)Lautstärkebereiche angewandt werden. In derartigen Ausführungsformen besitzen die abgestrahlten Testsignale unterschiedliche Lautstärken und Modifikationen in der Programmierung des Hörsystems zwei erfolgen jeweils für den betreffenden (Eingangs-)Lautstärkebereich. Die Durchführung von Datensammelsequenzen kann dabei z. B. für die verschiedenen (Eingangs-)Lautstärkebereiche gemeinsam erfolgen, die Auswertung und Modifikation der Programmierung jedoch zumindest teilweise separat.

## Patentansprüche

1. Verfahren zur Anpassung einer anwenderspezifischen Programmierung eines Hörsystems (2) mit Hilfe eines Hörsystemprogrammiergeräts (1), wobei das Verfahren umfasst:
a) Durchführen mehrerer Datensammelsequenzen mit unterschiedlichen Modifikationen der Programmierung des Hörsystems zum Erheben von Daten zum Differenzierungsvermögen eines Trägers des Hörsystems mit der jeweils aktuellen Programmierung des Hörsystems hinsichtlich ähnlich klingender gesprochener Buchstaben, Silben oder Phoneme, im Folgenden als schwierig differenzierbare Laute bezeichnet, wobei das Durchführen einer Datensammelsequenz mit der jeweils aktuellen Programmierung des Hörsystems umfasst:
- Erzeugen und Abstrahlen mindestens eines akustischen Testsignals in Form einer akustischen Übungsaufgabe mit mindestens zwei schwierig differenzierbaren Lauten, und
- Empfangen einer Reihe von Rückmeldungen des Trägers des Hörsystems (2) in Reaktion auf das mindestens eine Testsignal durch das Hörsystemprogrammiergerät (1), wobei die Rückmeldungen das Differenzierungsvermögen des Trägers hinsichtlich der mindestens zwei schwierig differenzierbaren Laute des Testsignals kodieren, wobei eine Rückmeldung dem korrekten Lösen der akustischen Übungsaufgabe entspricht, wenn sie einer entsprechenden Referenzrückmeldung entspricht, und eine Rückmeldung einem nicht korrekten Lösen der akustischen Übungsaufgabe entspricht, wenn sie einer alternativen Rückmeldung entspricht;
b) Durchführen einer Programmiersequenz im Anschluss an jede Datensammelsequenz, wobei die Programmiersequenz umfasst:
- Ermitteln eines Hörverständnisgrads in Bezug auf das Differenzierungsvermögen des Trägers für die empfangenen Rückmeldungen auf Basis der aktuellen Programmierung des Hörgeräts durch Vergleich der jeweiligen Rückmeldung mit der entsprechenden Referenz-Rückmeldung;
- Wenn für eine Reihe gleicher oder ähnlicher Testsignale der Anteil der nicht-korrekten Rückmeldungen einen Schwellwert überschreitet, Bestimmen einer modifizierten Programmierung des Hörsystems (2) in Abhängigkeit des ermittelten Hörverständnisgrads durch das Hörsystemprogrammiergerät (1), wobei das Bestimmen der modifizierten Programmierung unter Berücksichtigung einer Auswertung der empfangenen Rückmeldungen des Trägers des Hörsystems (2) erfolgt, indem die Verstärkung für einen differenzierenden Frequenzbereich oder für mehrere differenzierende Frequenzbereiche in Bezug auf die die mindestens zwei schwierig differenzierbaren Laute selektiv modifiziert wird während die Verstärkung für die übrigen Frequenzen bzw. Frequenzbereiche unverändert bleibt, wobei ein differenzierender Frequenzbereich Frequenzen umfasst, die entweder nur im Spektrum eines der Laute vorkommen, oder mit deutlich unterschiedlichem Amplituden- oder Leistungspegel in den Spektren beider Laute vorhanden sind;
- Übertragen der modifizierten Programmierung vom Hörsystemprogrammiergerät (2) zum Hörsystem (1) mittels einer Datenkommunikationsverbindung zwischen Hörsystemprogrammiergerät (1) und Hörsystem (2);
- Ändern der aktuellen Programmierung des Hörsystems (2) zur modifizierten Programmierung;
wobei beim Durchführen der Datensammelsequenzen mit anschließenden Programmiersequenzen Lautpaare aus unterschiedlichen schwierig differenzierbaren Lauten für das jeweilige Testsignal verwendet werden.

2. Verfahren nach Anspruch 1, wobei das mindestens eine Testsignal mindestens eine Zufallskomponente umfasst.

3. Verfahren nach einem der vorhergehenden Ansprüche, wobei das mindestens eine Testsignal mit der akustischen Übungsaufgabe mit mindestens zwei schwierig differenzierbaren Lauten mindestens eines von gesprochenen Ziffern, Zahlen, Lauten, Silben Phonemen, Wörtern, Wortgruppen, Sätzen umfasst.

4. Verfahren nach einem der vorhergehenden Ansprüche, wobei das Durchführen einer Anzahl von Datensammelsequenzen mit anschließender Programmiersequenz mit Lautpaaren aus unterschiedlichen schwierig differenzierbaren Lauten über mehrere Tage, insbesondere über mehrere aufeinander folgende Tage wiederholt wird.

5. Verfahren nach einem der vorhergehenden Ansprüche, wobei das Verfahren die Durchführung mehrerer Programmiersequenzen mit einer schrittweisen Anhebung der Verstärkung in Richtung einer Zielverstärkung umfasst.

6. Hörsystemprogrammiergerät (1), umfassend:
a) einen zur Erzeugung von akustischen Testsignalen ausgelegten Testsignalgenerator (11), wobei ein akustisches Testsignal die Form einer akustischen Übungsaufgabe mit mindestens zwei ähnlich klingenden gesprochenen Buchstaben, Silben oder Phonemen aufweist, im Folgenden als schwierig differenzierbare Laute bezeichnet;
b) mindestens eine mit dem Testsignalgenerator (11) operativ gekoppelte und zur akustischen Abstrahlung der Testsignale ausgelegte akustische Wiedergabeeinheit (12) und/oder eine mit dem Testsignalgenerator (11) operativ gekoppelte Übertragungseinheit zur Übertragung der Testsignale an eine Wiedergabeeinheit für die Durchführung mehrerer Datensammelsequenzen in Bezug auf schwierig differenzierbare Laute mit unterschiedlichen Modifikationen der Programmierung eines Hörsystems zum Erheben von Daten zum Differenzierungsvermögen eines Trägers des Hörsystems mit der jeweils aktuellen Programmierung des Hörsystems;
c) eine zum Empfang von Rückmeldungen eines Trägers des Hörsystems in Reaktion auf das mindestens eine Testsignal ausgelegte Eingabeeinheit (13), wobei die Rückmeldungen das Differenzierungsvermögen des Trägers hinsichtlich der mindestens zwei schwierig differenzierbaren Laute hinsichtlich des Testsignals kodieren, wobei eine Rückmeldung dem korrekten Lösen der akustischen Übungsaufgabe entspricht, wenn sie einer entsprechenden Referenzrückmeldung entspricht, und eine Rückmeldung einem nicht korrekten Lösen der akustischen Übungsaufgabe entspricht, wenn sie einer alternativen Rückmeldung entspricht;
d) eine zum Bestimmen einer modifizierten Programmierung eines Hörsystems (2) ausgelegte Programm-Modifikationseinheit (14), die weiterhin ausgelegt ist, die modifizierte Programmierung unter Berücksichtigung eines für die empfangenen Rückmeldungen ermittelten Hörverständnisgrads in Bezug auf das Differenzierungsvermögen auf Basis der aktuellen Programmierung des Hörgeräts zu bestimmen, wenn für eine Reihe gleicher oder ähnlicher Testsignale der Anteil der nicht-korrekten Rückmeldungen einen Schwellwert überschreitet, wobei das Bestimmen der modifizierten Programmierung unter Berücksichtigung einer Auswertung der empfangenen Rückmeldungen des Trägers des Hörsystems (2) durch Vergleich einer jeweiligen Rückmeldung mit der entsprechenden Referenz-Rückmeldung erfolgt, indem die Verstärkung für einen differenzierenden Frequenzbereich oder für mehrere differenzierende Frequenzbereiche in Bezug auf die die mindestens zwei schwierig differenzierbaren Laute selektiv modifiziert wird während die Verstärkung für die übrigen Frequenzen bzw. Frequenzbereiche unverändert bleibt, wobei ein differenzierender Frequenzbereich Frequenzen umfasst, die entweder nur im Spektrum eines der Laute vorkommen, oder mit deutlich unterschiedlichem Amplituden- oder Leistungspegel in den Spektren beider Laute vorhanden sind;
e) einen mit der Programm-Modifikationseinheit (14) operativ gekoppelten lernfähigen Konfigurationsspeicher (16) zur Speicherung der modifizierten Programmierung des Hörsystems (2);
f) eine zur Datenkommunikation mit dem Hörsystem (2) und zur Übertragung der modifizierten Programmierung an das Hörsystem ausgelegte Kommunikationseinheit (15).

7. Computerprogrammprodukt zum Konfigurieren eines Hörsystems umfassend Befehle, die bewirken, dass das Hörsystemprogrammiergerät des Anspruchs 6 die folgenden Schritte ausführt:
a) Durchführen mehrerer Datensammelsequenzen mit unterschiedlichen Modifikationen der Programmierung des Hörsystems zum Erheben von Daten zum Differenzierungsvermögen eines Trägers des Hörsystems mit der jeweils aktuellen Programmierung des Hörsystems hinsichtlich ähnlich klingender gesprochener Buchstaben, Silben oder Phoneme, im Folgenden als schwierig differenzierbare Laute bezeichnet, wobei das Durchführen einer Datensammelsequenz mit der jeweils aktuellen Programmierung des Hörsystems umfasst:
- Erzeugen und Abstrahlen mindestens eines akustischen Testsignals in Form einer akustischen Übungsaufgabe mit mindestens zwei schwierig differenzierbaren Lauten, und
- Empfangen einer Reihe von Rückmeldungen des Trägers des Hörsystems (2) in Reaktion auf das mindestens eine Testsignal durch das Hörsystemprogrammiergerät (1), wobei die Rückmeldungen das Differenzierungsvermögen des Trägers hinsichtlich der mindestens zwei schwierig differenzierbaren Laute des Testsignals kodieren wobei eine Rückmeldung dem korrekten Lösen der akustischen Übungsaufgabe entspricht, wenn sie einer entsprechenden Referenzrückmeldung entspricht, und eine Rückmeldung einem nicht korrekten Lösen der akustischen Übungsaufgabe entspricht, wenn sie einer alternativen Rückmeldung entspricht;
b) Durchführen einer Programmiersequenz im Anschluss an jede Datensammelsequenz, wobei die Programmiersequenz umfasst:
- Ermitteln eines Hörverständnisgrads in Bezug auf das Differenzierungsvermögen des Trägers für die empfangenen Rückmeldungen auf Basis der aktuellen Programmierung des Hörgeräts durch Vergleich der jeweiligen Rückmeldung mit der entsprechenden Referenz-Rückmeldung;
- Wenn für eine Reihe gleicher oder ähnlicher Testsignale der Anteil der nicht-korrekten Rückmeldungen einen Schwellwert überschreitet, Bestimmen einer modifizierten Programmierung des Hörsystems (2) in Abhängigkeit des ermittelten Hörverständnisgrads durch das Hörsystemprogrammiergerät (1), wobei das Bestimmen der modifizierten Programmierung unter Berücksichtigung einer Auswertung der empfangenen mindestens einen Rückmeldung des Trägers des Hörsystems (2) erfolgt, indem die Verstärkung für einen differenzierenden Frequenzbereich oder für mehrere differenzierende Frequenzbereiche in Bezug auf die die mindestens zwei schwierig differenzierbaren Laute selektiv modifiziert wird während die Verstärkung für die übrigen Frequenzen bzw. Frequenzbereiche unverändert bleibt, wobei ein differenzierender Frequenzbereich Frequenzen umfasst, die entweder nur im Spektrum eines der Laute vorkommen, oder mit deutlich unterschiedlichem Amplituden- oder Leistungspegel in den Spektren beider Laute vorhanden sind;
- Übertragen der modifizierten Programmierung vom Hörsystemprogrammiergerät (2) zum Hörsystem (1) mittels einer Datenkommunikationsverbindung zwischen Hörsystemprogrammiergerät (1) und Hörsystem (2);
- Ändern der aktuellen Programmierung des Hörsystems (2) zur modifizierten Programmierung;
wobei beim Durchführen der Datensammelsequenzen mit anschließenden Programmiersequenzen Lautpaare aus unterschiedlichen schwierig differenzierbaren Lauten für das jeweilige Testsignal verwendet werden.

8. Computerprogrammprodukt nach Anspruch 7, wobei das mindestens eine Testsignal mindestens eine Zufallskomponente umfasst.

9. Computerprogrammprodukt nach Anspruch 7 oder 8 welches die Computereinheit weiterhin dazu veranlasst, den folgenden Schritt auszuführen:
Speichern der modifizierten Programmierung in einem lernfähigen Konfigurationsspeicher (16).

10. Computerprogrammprodukt nach einem der Ansprüche 7 bis 8, wobei das mindestens eine Testsignal mit der akustischen Übungsaufgabe mit mindestens zwei schwierig differenzierbaren Lauten mindestens eines von gesprochenen Ziffern, Zahlen, lauten, Silben Phonemen, Wörtern, Wortgruppen, Sätzen umfasst.

## Claims

1. A method for adjusting user-specific programming of a hearing system (2) by means of a hearing system programming device (1), wherein the method comprises:
a) carrying out a plurality of data collection sequences having different modifications to the programming of the hearing system for gathering data on the ability to differentiate of a wearer of the hearing system using the current programming of the hearing system with regard to similar-sounding spoken letters, syllables, or phonemes, referred to in the following as difficult to differentiate sounds, wherein carrying out a data collection sequence using the current programming of the hearing system comprises:
- generating and emitting at least one acoustic test signal in the form of an acoustic exercise that has at least two difficult to differentiate sounds, and
- receiving a series of responses from the wearer of the hearing system (2) in response to the at least one test signal by the hearing system programming device (1), the responses coding the wearer's ability to differentiate with regard to the at least two difficult to differentiate sounds of the test signal, wherein one response corresponds to the correct solution to the acoustic exercise when it corresponds to a corresponding reference response, and one response corresponds to an incorrect solution to the acoustic exercise when it corresponds to an alternative response;
b) carrying out a programming sequence following each data collection sequence, wherein the programming sequence comprises:
- ascertaining a level of hearing comprehension in relation to the wearer's ability to differentiate for the received responses on the basis of the current programming of the hearing device by comparing the response in question with the corresponding reference response;
- if, for a series of identical or similar test signals, the proportion of incorrect responses exceeds a threshold value, determining modified programming of the hearing system (2) on the basis of the ascertained level of hearing comprehension by the hearing system programming device (1), wherein determining the modified programming takes into account an evaluation of the received responses from the wearer of the hearing system (2) by the amplification being selectively modified for a differentiating frequency range or a plurality of differentiating frequency ranges in relation to the at least two difficult to differentiate sounds while the amplification for the other frequencies or frequency ranges remains unchanged, wherein a differentiating frequency range comprises frequencies that are either only within the spectrum of one of the sounds or are present within the spectra of both sounds but with significantly different amplitude or power levels;
- transmitting the modified programming from the hearing system programming device (2) to the hearing system (1) by means of a data communication link between the hearing system programming device (1) and the hearing system (2);
- changing the current programming of the hearing system (2) to the modified programming;
wherein, when carrying out the data collection sequences with subsequent programming sequences, sound pairs of different difficult to differentiate sounds are used for the test signal in question.

2. The method according to claim 1, wherein the at least one test signal comprises at least one random component.

3. The method according to any of the preceding claims, wherein the at least one test signal having the acoustic exercise having at least two difficult to differentiate sounds comprises at least one of spoken digits, numbers, sounds, syllables, phonemes, words, word groups, sentences.

4. The method according to any of the preceding claims, wherein a number of data collection sequences with a subsequent programming sequence having sound pairs of different difficult to differentiate sounds is carried out repeatedly over a plurality of days, in particular over a plurality of consecutive days.

5. The method according to any of the preceding claims, wherein the method comprises carrying out a plurality of programming sequences while gradually increasing the amplification toward a target amplification.

6. A hearing system programming device (1), comprising:
a) a test signal generator (11) configured to generate acoustic test signals, wherein an acoustic test signal takes the form of an acoustic exercise having at least two similar-sounding spoken letters, syllables, or phonemes, referred to in the following as difficult to differentiate sounds;
b) at least one acoustic reproduction unit (12) that is operatively coupled to the test signal generator (11) and is configured for the acoustic emission of the test signals and/or and a transmission unit that is operatively coupled to the test signal generator (11) for the transmission of the test signals to a reproduction unit for carrying out a plurality of data collection sequences in relation to difficult to differentiate sounds with different modifications to the programming of a hearing system for gathering data on the ability to differentiate of a wearer of the hearing system using the current programming of the hearing system;
c) an input unit (13) configured to receive responses from a wearer of the hearing system in response to the at least one test signal, the responses coding the wearer's ability to differentiate with regard to the at least two difficult to differentiate sounds with regard to the test signal, wherein one response corresponds to the correct solution to the acoustic exercise when it corresponds to a corresponding reference response, and one response corresponds to an incorrect solution to the acoustic exercise when it corresponds to an alternative response;
d) a program modification unit (14) configured to determine modified programming of a hearing system (2), which unit is further configured to determine the modified programming taking into account a level of hearing comprehension ascertained for the received responses in relation to the ability to differentiate on the basis of the current programming of the hearing device, if, for a series of identical or similar test signals, the proportion of incorrect responses exceeds a threshold value, wherein determining the modified programming takes into account an evaluation of the received responses from the wearer of the hearing system (2) by comparing the response in question with the corresponding reference response, by the amplification being selectively modified for a differentiating frequency range or a plurality of differentiating frequency ranges in relation to the at least two difficult to differentiate sounds while the amplification for the other frequencies or frequency ranges remains unchanged, wherein a differentiating frequency range comprises frequencies that are either only within the spectrum of one of the sounds or are present within the spectra of both sounds but with significantly different amplitude or power levels;
e) a trainable configuration memory (16) that is operatively coupled to the program modification unit (14) for storing the modified programming of the hearing system (2);
f) a communication unit (15) configured for data communication with the hearing system (2) and for transmission of the modified programming to the hearing system.

7. A computer program product for configuring a hearing system comprising commands that prompt the hearing system programming device from claim 6 to perform the following steps:
a) carrying out a plurality of data collection sequences having different modifications to the programming of the hearing system for gathering data on the ability to differentiate of a wearer of the hearing system using the current programming of the hearing system with regard to similar-sounding spoken letters, syllables, or phonemes, referred to in the following as difficult to differentiate sounds, wherein carrying out a data collection sequence using the current programming of the hearing system comprises:
- generating and emitting at least one acoustic test signal in the form of an acoustic exercise that has at least two difficult to differentiate sounds, and
- receiving a series of responses from the wearer of the hearing system (2) in response to the at least one test signal by the hearing system programming device (1), the responses coding the wearer's ability to differentiate with regard to the at least two difficult to differentiate sounds of the test signal, wherein one response corresponds to the correct solution to the acoustic exercise when it corresponds to a corresponding reference response, and one response corresponds to an incorrect solution to the acoustic exercise when it corresponds to an alternative response;
b) carrying out a programming sequence following each data collection sequence, wherein the programming sequence comprises:
- ascertaining a level of hearing comprehension in relation to the wearer's ability to differentiate for the received responses on the basis of the current programming of the hearing device by comparing the response in question with the corresponding reference response;
- if, for a series of identical or similar test signals, the proportion of incorrect responses exceeds a threshold value, determining modified programming of the hearing system (2) on the basis of the ascertained level of hearing comprehension by the hearing system programming device (1), wherein determining the modified programming takes into account an evaluation of the received at least one response from the wearer of the hearing system (2) by the amplification being selectively modified for a differentiating frequency range or a plurality of differentiating frequency ranges in relation to the at least two difficult to differentiate sounds while the amplification for the other frequencies or frequency ranges remains unchanged, wherein a differentiating frequency range comprises frequencies that are either only within the spectrum of one of the sounds or are present within the spectra of both sounds but with significantly different amplitude or power levels;
- transmitting the modified programming from the hearing system programming device (2) to the hearing system (1) by means of a data communication link between the hearing system programming device (1) and the hearing system (2);
- changing the current programming of the hearing system (2) to the modified programming;
wherein, when carrying out the data collection sequences with subsequent programming sequences, sound pairs of different difficult to differentiate sounds are used for the test signal in question.

8. The computer program product according to claim 7, wherein the at least one test signal comprises at least one random component.

9. The computer program product according to claim 7 or 8, which also causes the computer unit to perform the following step: storing the modified programming in a trainable configuration memory (16).

10. The computer program product according to any of claims 7 to 8, wherein the at least one test signal having the acoustic exercise having at least two difficult to differentiate sounds comprises at least one of spoken digits, numbers, sounds, syllables, phonemes, words, word groups, sentences.

## Revendications

1. Procédé pour adapter une programmation spécifique de l'utilisateur d'un système auditif (2) à l'aide d'un appareil (1) de programmation de système auditif, le procédé comprenant :
a) la réalisation de plusieurs séquences de collecte de données avec différentes modifications de la programmation du système auditif pour recueillir des données sur la capacité de différenciation d'un porteur du système auditif avec la programmation actuelle du système auditif en ce qui concerne des lettres, des syllabes ou des phonèmes parlés à consonance similaire, appelés ci-après sons difficilement différentiables, où la réalisation d'une séquence de collecte de données avec la programmation actuelle du système auditif comprend :
- la génération et l'émission d'au moins un signal de test acoustique sous la forme d'un exercice d'entraînement acoustique avec au moins deux sons difficilement différentiables, et
- la réception d'une série de retours d'informations de la part du porteur du système auditif (2) en réponse audit au moins un signal de test par l'appareil (1) de programmation de système auditif, où les retours d'informations codent la capacité de différentiation du porteur en ce qui concerne les au moins deux sons difficilement différentiables du signal de test, où un retour d'informations correspond à la résolution correcte de l'exercice d'entraînement acoustique lorsqu'il correspond à un retour d'informations de référence correspondant, et un retour d'informations correspond à une résolution incorrecte de l'exercice d'entraînement acoustique lorsqu'il correspond à un retour d'informations alternatif ;
b) la réalisation d'une séquence de programmation à la suite de chaque séquence de collecte de données, où la séquence de programmation comprend :
- l'estimation d'un niveau de compréhension auditive par rapport à la capacité de différentiation du porteur pour les retours d'informations reçus sur la base de la programmation actuelle de l'appareil auditif, par comparaison de chaque retour d'informations avec le retour d'informations de référence correspondant ;
- si la proportion de retours d'informations incorrects dépasse une valeur seuil pour une série de signaux de test identiques ou similaires, la détermination d'une programmation modifiée du système auditif (2) en fonction du niveau de compréhension auditive estimé par l'intermédiaire de l'appareil (1) de programmation de système auditif, où la détermination de la programmation modifiée est effectuée en tenant compte d'une évaluation des retours d'informations reçus du porteur du système auditif (2), en modifiant sélectivement le gain pour une plage de fréquences de différentiation ou pour plusieurs plages de fréquences de différentiation par rapport aux au moins deux sons difficilement différentiables, tandis que le gain pour les fréquences ou plages de fréquences restantes reste inchangé, où une plage de fréquences de différentiation comprend des fréquences qui soit n'apparaissent que dans le spectre de l'un des sons, soit sont présentes dans les spectres des deux sons avec des niveaux d'amplitude ou de puissance sensiblement différents ;
- transmission de la programmation modifiée de l'appareil (1) de programmation de système auditif au système auditif (2) au moyen d'une liaison de communications de données entre l'appareil (1) de programmation de système auditif et le système auditif (2) ;
- changement de la programmation actuelle du système auditif (2) pour la programmation modifiée ;
dans lequel, lors de la réalisation des séquences de collecte de données suivies de séquences de programmation, des paires de sons de sons différents et difficilement différentiables sont utilisées pour chaque signal de test.

2. Procédé selon la revendication 1, dans lequel l'au moins un signal de test comprend au moins une composante aléatoire.

3. Procédé selon l'une des revendications précédentes, dans lequel l'au moins un signal de test avec l'exercice d'entraînement acoustique avec au moins deux sons difficilement différentiables comprend au moins l'un, prononcé, parmi des chiffres, des nombres, des sons, des syllabes, des phonèmes, des mots, des groupes de mots, des phrases.

4. Procédé selon l'une des revendications précédentes, dans lequel la réalisation d'une pluralité de séquences de collecte de données suivies d'une séquence de programmation avec des paires de sons de sons différents et difficilement différentiables est répétée sur plusieurs jours, en particulier sur plusieurs jours consécutifs.

5. Procédé selon l'une des revendications précédentes, le procédé comprenant la réalisation de plusieurs séquences de programmation avec une augmentation progressive du gain vers un gain cible.

6. Appareil (1) de programmation de système auditif, comprenant :
a) un générateur (11) de signaux de test conçu pour générer des signaux de test acoustiques, où un signal de test acoustique se présente sous la forme d'un exercice d'entraînement acoustique avec au moins deux lettres, syllabes ou phonèmes parlés à consonance similaire, appelés ci-après sons difficilement différentiables ;
b) au moins une unité (12) de reproduction acoustique couplée fonctionnellement au générateur (11) de signaux de test et conçue pour l'émission acoustique des signaux de test et/ou une unité de transmission couplée fonctionnellement au générateur (11) de signaux de test pour la transmission des signaux de test à une unité de reproduction pour la réalisation de plusieurs séquences de collecte de données concernant des sons difficilement différentiables avec différentes modifications de la programmation d'un système auditif pour recueillir des données concernant la capacité de différenciation d'un porteur du système auditif avec la programmation actuelle respective du système auditif ;
c) une unité d'entrée (13) adaptée pour recevoir des retours d'informations d'un porteur du système auditif en réponse audit au moins un signal de test, où les retours d'informations codent la capacité de différentiation du porteur en ce qui concerne les au moins deux sons difficilement différentiables du signal de test, où un retour d'informations correspond à la résolution correcte de l'exercice d'entraînement acoustique lorsqu'il correspond à un retour d'informations de référence correspondant, et un retour d'informations correspond à une résolution incorrecte de l'exercice d'entraînement acoustique lorsqu'il correspond à un retour d'informations alternatif ;
d) une unité (14) de modification de programme conçue pour déterminer une programmation modifiée d'un système auditif (2), qui est en outre conçue pour déterminer la programmation modifiée en tenant compte d'un degré de compréhension auditive déterminé pour l'au moins un retour d'informations reçu sur la base de la programmation actuelle de l'appareil auditif, si la proportion de retours d'informations incorrects dépasse une valeur seuil pour une série de signaux de test identiques ou similaires, où la détermination de la programmation modifiée est effectuée en tenant compte d'une évaluation des retours d'informations reçus du porteur du système auditif (2), en modifiant sélectivement le gain pour une plage de fréquences de différentiation ou pour plusieurs plages de fréquences de différentiation par rapport aux au moins deux sons difficilement différentiables, tandis que le gain pour les fréquences ou plages de fréquences restantes reste inchangé, où une plage de fréquences de différentiation comprend des fréquences qui soit n'apparaissent que dans le spectre de l'un des sons, soit sont présentes dans les spectres des deux sons avec des niveaux d'amplitude ou de puissance sensiblement différents ;
e) une mémoire (16) de configuration d'apprentissage couplée fonctionnellement à l'unité (14) de modification de programme pour stocker la programmation modifiée du système auditif (2) ;
f) une unité (15) de communication conçue pour la communication de données avec le système auditif (2) et pour transmettre la programmation modifiée au système auditif.

7. Produit-programme informatique destiné à configurer un système auditif (2), comprenant des instructions qui, lorsqu'elles sont exécutées, amènent l'appareil de programmation de système auditif selon la revendication 6 à réaliser les étapes suivantes :
a) réalisation de plusieurs séquences de collecte de données avec différentes modifications de la programmation du système auditif pour recueillir des données sur la capacité de différenciation d'un porteur du système auditif avec la programmation actuelle du système auditif en ce qui concerne des lettres, des syllabes ou des phonèmes parlés à consonance similaire, appelés ci-après sons difficilement différentiables, où la réalisation d'une séquence de collecte de données avec la programmation actuelle du système auditif comprend :
- la génération et l'émission d'au moins un signal de test acoustique sous la forme d'un exercice d'entraînement acoustique avec au moins deux sons difficilement différentiables, et
- réception d'une série de retours d'informations de la part du porteur du système auditif (2) en réponse audit au moins un signal de test par l'appareil (1) de programmation de système auditif, où les retours d'informations codent la capacité de différentiation du porteur en ce qui concerne les au moins deux sons difficilement différentiables du signal de test, où un retour d'informations correspond à la résolution correcte de l'exercice d'entraînement acoustique lorsqu'il correspond à un retour d'informations de référence correspondant, et un retour d'informations correspond à une résolution incorrecte de l'exercice d'entraînement acoustique lorsqu'il correspond à un retour d'informations alternatif ;
b) réalisation d'une séquence de programmation à la suite de chaque séquence de collecte de données, où la séquence de programmation comprend :
- la détermination d'un niveau de compréhension auditive par rapport à la capacité de différentiation du porteur pour les retours d'informations reçus sur la base de la programmation actuelle de l'appareil auditif, par comparaison de chaque retour d'informations avec le retour d'informations de référence correspondant ;
- si la proportion de retours d'informations incorrects dépasse une valeur seuil pour une série de signaux de test identiques ou similaires, la détermination d'une programmation modifiée du système auditif (2) en fonction du niveau de compréhension auditive estimé par l'intermédiaire de l'appareil (1) de programmation de système auditif, où la détermination de la programmation modifiée est effectuée en tenant compte d'une évaluation de l'au moins un retour d'informations reçu du porteur du système auditif (2), en modifiant sélectivement le gain pour une plage de fréquences de différentiation ou pour plusieurs plages de fréquences de différentiation par rapport aux au moins deux sons difficilement différentiables, tandis que le gain pour les fréquences ou plages de fréquences restantes reste inchangé, où une plage de fréquences de différentiation comprend des fréquences qui soit n'apparaissent que dans le spectre de l'un des sons, soit sont présentes dans les spectres des deux sons avec des niveaux d'amplitude ou de puissance sensiblement différents ;
- transmission de la programmation modifiée de l'appareil (1) de programmation de système auditif au système auditif (1) au moyen d'une liaison de communications de données entre l'appareil (1) de programmation de système auditif et le système auditif (2) ;
- changement de la programmation actuelle du système auditif (2) pour la programmation modifiée ;
dans lequel, lors de la réalisation des séquences de collecte de données suivies de séquences de programmation, des paires de sons de sons différents et difficilement différentiables sont utilisées pour chaque signal de test.

8. Produit-programme informatique selon la revendication 7, dans lequel l'au moins un signal de test comprend au moins une composante aléatoire.

9. Produit-programme informatique selon la revendication 7 ou 8, qui amène en outre l'unité informatique à exécuter l'étape suivante :
stockage de la programmation modifiée dans une mémoire (16) de configuration d'apprentissage.

10. Produit-programme informatique selon l'une des revendications 7 à 8, dans lequel l'au moins un signal de test avec l'exercice d'entraînement acoustique avec au moins deux sons difficilement différentiables comprend au moins l'un, prononcé, parmi des chiffres, des nombres, des sons, des syllabes, des phonèmes, des mots, des groupes de mots, des phrases.
